# EUROPEAN PATENT APPLICATION

(11) **EP 4 541 375 A1**
(43) Date of publication of application: **23.04.2025**
(21) Application number: 23823198.9
(22) Date of filing: 14.06.2023
(51) Int. Cl.: A61K 47/60, C07K 5/08, A61P 35/00

(54) **CONJUGATE OF ANTI-CTLA-4 ANTIBODY ACTIVATED BY TUMOR MICROENVIRONMENT AND USE THEREOF**

(30) Priority: 16.06.2022 CN 202210721342
(71) Applicant: Yafei Shanghai Biology Medicine Science & Technology Co., Ltd., Shanghai 201203 (CN)
(72) Inventor: LIU, Chen, Shanghai 201203 (CN); LIU, Yuan, Shanghai 201203 (CN)
(74) Representative: IPLodge bv
(86) International application number: PCT/CN2023/100258
(87) International publication number: WO 2023/241632

(57) **Abstract**

Provided are a conjugate of an anti-CTLA-4 antibody activated by a tumor microenvironment and an application thereof. The conjugate of the present invention has the following structure: R1-R2-R3-L-R4-S-cys-R5, wherein R1, R2, R3, L, R4, and R5 each represent structures such as different functional groups, linkers, chemical bonds, and CTLA-4 antibodies. The conjugate of the present invention is a dual-activated conjugate, which can improve the targeting efficacy of the anti-CTLA-4 antibody while overcoming the drug resistance of the antibody and reducing toxicity.

## Description

### TECHNICAL FIELD

The present invention relates to a conjugate of an anti-CTLA-4 antibody activated by a tumor microenvironment and an application thereof.

### BACKGROUND

Tumor immunotherapy is one of the breakthroughs in the field of tumor treatment in recent years, making long-term survival possible for some patients. Tumor immunotherapy includes tumor immune checkpoint antibodies, bispecific T cell engager (bsTCE), chimeric antigen receptor T cells (CAR-T), oncolytic viruses and so on. Wherein, CTLA-4 antibody is the first immune checkpoint inhibitor antibody approved by FDA. PD-1 antibody is another immune checkpoint inhibitor with remarkable clinical efficacy. CTLA-4 antibody, PD-1 antibody and their combination therapy have achieved very superior curative effects in clinical trials of cancers including melanoma, non-small cell lung cancer, lymphoma and liver cancer, and some therapies have become first-line therapies.

Since immune checkpoint antibodies remove the inhibitory signaling pathway of T cells, they often lead to non-specific activation of T cells in non-tumor target organs, resulting in immune-related adverse events (irAEs). However, the response rate of treatment with single immune checkpoint antibodies is often limited, usually only about 20-30%. In order to improve the response rate of patients, combined immunotherapy such as PD-1 + CTLA-4 dual-target immunotherapy is needed, which however significantly enhances the effect of irAEs. For example, in the clinical study of CTLA-4 combined with PD-1 in the treatment of malignant melanoma, the overall response rate is 58%, which is significantly improved compared with CTLA-4 alone (19%) or PD-1 alone (44%). However, with the improvement of curative effect, more serious treatment-related adverse events (TRAEs) are also observed clinically, wherein grade 3 to 4 TRAEs increased to 55%, and the incidence rate is significantly increased compared with CTLA-4 alone (27%) or PD-1 alone (16%). Common irAEs include hypophysitis, hepatitis, colitis, rash and diarrhea, etc. These serious irAEs can even lead to the death of subjects.

CTLA-4 is an inhibitory receptor expressed on the surface of T cells, its mechanism lies in competitively binding to B7.1 (CD80) and B7.2 (CD86) with CD28, resulting in T cells not being over-activated after receiving antigens presented by MHC. When the inhibitory signal is removed by CTLA-4 antibodies such as Ipilimumab, the activation and proliferation of T cells after being stimulated by MHC-antigen complexes increase, thereby enhancing the tumor immune effect mediated by T cells. Meanwhile, recent research has discovered that CTLA-4 is highly expressed on the surface of Treg cells, CTLA-4 antibodies with Fc effector functions, such as Ipilimumab, can eliminate Treg cells within tumors through IgG1 Fc-mediated ADCC (Antibody-Dependent Cellular Cytotoxicity) effects, thereby eliminating the suppressive microenvironment of the tumor and enhancing the anti-tumor immune response.

As mentioned above, whether it is based on the removal of T cell activation inhibitory signals or the killing of Treg cells through the ADCC effect, both effects promote autoimmune reactions in normal organs, leading to the occurrence of irAEs. Therefore, there is an urgent need in clinical practice to develop safer anti-CTLA-4 antibodies to limit their immune-enhancing effects to tumors and not cause autoimmune toxicity to normal tissues.

### SUMMARY

To solve the above technical problem, the present invention provides a conjugate of an anti-CTLA-4 antibody that is activated in the tumor microenvironment, which has the following structure:

R1-R2-R3-L-R4-S-cys-R5

wherein,
R5 represents an anti-CTLA-4 antibody in which one or more amino acid residues are mutated to cysteine;
cys represents the cysteine residue of R5;
S represents the S atom in the cysteine residue;
R1 is a functional group that blocks the binding of R5 to its antigen, ligand or receptor;
R2 is a linker that can be hydrolyzed and cleaved by one or more proteases in the pathological microenvironment;
R3 is a linker that provides space for protease cleavage;
L is a chemical bond activated under acidic conditions in the pathological microenvironment;
R4 is a group covalently linked to R5 through the sulfur atom on cysteine in R5, which can restore, maintain or improve the binding ability of R5 to its antigen, ligand and receptor after R1-R2-R3-L- is cleaved.

The present invention provides a pharmaceutical composition comprising a conjugate as described herein.

The present invention provides a method for treating or preventing tumors, comprising providing a therapeutically or prophylactically effective amount of a conjugate according to any of the embodiments herein to a subject in need.

The present invention also provides the use of the conjugate described herein in the preparation of a medicament for treating or preventing tumors.

The present invention also provides a conjugate according to any of the embodiments herein for treatment or prophylaxis.

### DESCRIPTION OF DRAWINGS

Figure 1: Conjugation efficiency of Ipi-L-3 with different compounds.
Figure 2: Enzyme-linked immunosorbent assay (ELISA) characterization of the binding ability of Ipi-L-3 TMEAbody.
Figure 3: Acid-mediated activation of Ipi-L-3 TMEAbody.
Figure 4: ELISA characterization of the acid activation properties of Ipi-L-3 TMEAbody.
Figure 5: Ipi-L-3 +CP23 TMEAbody can be activated by legumain.
Figure 6: ELISA characterization of legumain-mediated activation of Ipi-L-3+CP23 TMEAbody.
Figure 7: Schematic diagram of Ipi-L-3 TMEAbody activated by legumain or low pH environment.
Figure 8: (A) Characterization of the conjugation of Ipi-L-3 and R4-4 by enzyme-linked immunosorbent assay; (B) Characterization of the conjugation of Ipi-L-3 and CP31 by enzyme-linked immunosorbent assay.
Figure 9: Polyacrylamide gel electrophoresis diagram of TMEAbody after extremely low pH treatment.
Figure 10: Characterization of TMEAbody after treatment with extremely low pH conditions by enzyme-linked immunosorbent assay.
Figure 11: Tumor permeability of different TMEAbodies.
Figure 12: In vivo efficacy of Ipi-L-3 + CP23 TMEAbody in hCTLA-4 gene knock-in MC38 mouse model.
Figure 13: Ipi-L-3 + CP23 increases plasma half-life and AUC.
Figure 14: Ipi-L-3 TMEAbody shows a lower peripheral T cell activation effect.
Figure 15: Ipi-L-3 + CP23 produces lower systemic toxicity in the GvHD model.
Figure 16: Ipi-L-3 TMEAbody shows lower immunogenicity in mice.
Figure 17: The number of Tregs after treatment with Ipi-L-3 + CP23 TMEAbody analyzed by FACS.

### DETAILED DESCRIPTION

It should be understood that within the scope of the disclosure of the present invention, each technical feature involved can be combined with the technical features mentioned later, including the technical features enumerated in the embodiments, to form a preferred technical solution for solving technical problems. Moreover, the disclosure by the present invention is not limited to the specific embodiments described, so there can be one or more variations. It should also be understood that, unless otherwise defined, all technical and scientific terms used herein have the same meaning as commonly understood by those of ordinary skills in the field to which the present invention belongs. Although any other methods and materials similar or equivalent to those described herein can also be used in the practice or testing of the present invention, the methods and materials described herein are preferred. All publications mentioned herein are incorporated herein by reference to disclose and describe the methods and/or materials related to the cited publications.

It should be noted that, as used herein and the appended claims, the singular forms "one," "a," "and" and "the" include the plural unless the context clearly dictates otherwise. It should also be noted that claims can be drafted to exclude any optional elements. Therefore, this statement is intended to serve as a prior basis for the use of special terms such as "alone", "only" and the like related to the narration of claim elements or the use of "negative" limitations.

One of the purposes of the present invention is to provide a modified anti-CTLA-4 antibody conjugate which is only activated in a pathological microenvironment (such as in a tumor microenvironment or an inflammatory site) to release an anti-CTLA-4 antibody with equivalent or enhanced binding force to its ligand, thereby improving the targeting efficacy of biomolecules and simultaneously overcoming drug resistance and reducing toxicity.

In the present invention, the modified anti-CTLA-4 antibody is a conjugate which contains an anti-CTLA-4 antibody and a functional part covalently linked to the antibody, i.e., R1-R2-R3-L-R4.

In the disclosure herein, the term "antibody" is used in its broadest sense, encompassing monoclonal antibodies, polyclonal antibodies, dimers, multimers, multispecific antibodies (such as bispecific antibodies), and antibody fragments, as long as they exhibit the desired biological activity (Miller et al., (2003), Jour. of Immunology, 170: 4854-4861). In the context and the figures, the abbreviation "Ab" is used to represent the antibody.

The basic structural unit of an antibody consists of a tetramer made up of two identical pairs of polypeptide chains, each pair comprising one light chain and one heavy chain. The N-terminal portion of each chain includes a variable region composed of approximately 100 to 110 or more amino acids, primarily responsible for antigen recognition. The C-terminal portion of each chain defines the constant region, which is mainly responsible for effector functions. The heavy chain variable region (VH) and the light chain variable region (VL) each contain three complementarity-determining regions (CDRs), known as HCDR1, HCDR2, HCDR3, and LCDR1, LCDR2, and LCDR3. These six CDRs form the antigen-binding site of the antibody. The remaining amino acids in the variable region are relatively conserved and are referred to as framework regions (FRs). Both VH and VL have four framework regions, designated as FR1, FR2, FR3, and FR4.

Antibodies can be derived from mice, humans, or other sources, and can be humanized antibodies or chimeric antibodies. The immunoglobulins disclosed in the present invention can be of any class (such as IgG, IgE, IgM, IgD, and IgA), subclass (such as IgG1, IgG2, IgG3, IgG4, IgA1, and IgA2), or isotype of immunoglobulin molecules. Immunoglobulins can come from any species, such as humans, mice, or rabbits.

Antibody fragments comprise a portion of the full-length antibody, typically including the antigen-binding site or variable region of the antibody. Preferably, the antibody fragment is a functional fragment that retains the antigen-binding capability of the intact antibody, meaning the antibody fragment is the antigen-binding fragment of the antibody. Examples of antibody fragments or functional fragments include Fab, Fab', F(ab')2, and Fv fragments; dimers; linear antibodies; and single-chain antibody molecules (scFv); etc.

In the present invention, one or more (for example, five or fewer, or three or fewer) amino acids at suitable positions in the amino acid sequence of the anti-CTLA-4 antibody are mutated to cysteine, and then covalently linked to the functional part (R1-R2-R3-L-R4) of the present invention through the sulfhydryl group of this cysteine. For example, one or two amino acids of interest in the anti-CTLA-4 antibody of interest are mutated to cysteine for conjugation to the functional part. The mutation position can exist in the complementarity determining region or non-complementarity determining region of the variable region. Preferably, the mutation is a substitution mutation. More preferably, the mutation occurs in the light chain variable region of the anti-CTLA-4 antibody. Generally, mutants can be prepared and then tested for their binding activity to the corresponding antigen. If the mutant retains 70% or more, preferably 80% or more, more preferably 90% or more of the binding activity compared to the wild-type antibody, it is considered that the amino acid residue at the mutation position can be mutated to cysteine for covalent linking to the functional part. Alternatively, in some embodiments, if the conjugate produced by linking the mutant to R4 described herein retains 80% or more, preferably 90% or more, more preferably 95% or more of the binding activity, it is considered that the amino acid residue at this position can be mutated to cysteine for covalent linking to the functional part.

Various anti-CTLA-4 antibodies can be used in the present invention. The preferred anti-CTLA-4 antibody is Ipilimumab, and the exemplary amino acid sequences of its heavy chain and light chain are shown as SEQ ID NO:1 and 2 respectively.

Generally, one or more of the amino acids G, A, S, T, L, I, F, E, K, D, and Y in the CDR of the light chain variable region of the anti-CTLA-4 antibody, more preferably one or more of G, A, S, T, L, I, K, and Y, and even more preferably one or more of G, A, T, L, S, and Y, can be mutated to cysteine. In some embodiments, one or more of the amino acids G, A, S, T, L, I, F, E, K, D, and Y in the CDR of the heavy chain variable region of the anti-CTLA-4 antibody, more preferably one or more of G, A, S, T, L, I, K, and Y, and even more preferably one or more of G, A, T, L, S, and Y, can be mutated to cysteine. If the mutation occurs in a non-CDR region of the light or heavy chain variable region of the anti-CTLA-4 antibody, then one or more of the amino acids G, A, S, T, L, I, F, E, K, D, and Y in the non-CDR region of the light or heavy chain variable region, preferably one or more of G, A, S, T, K, I, Y, and L, and more preferably one or more of G, A, T, Y, and S, can be mutated to cysteine. In some embodiments, one or more of the S, T, L, I, F, E, K, D, N, Q, R, and Y residues in the non-complementary determining regions (e.g., FR1, FR2, or FR3) of the light or heavy chain variable region of the anti-CTLA-4 antibody can be mutated to cysteine. In some embodiments, substitution mutations can be introduced into one or more of the following conserved sites: Gln3, Ser7, Ser25, Glu46, Thr69, and Asp73 in the non-complementary determining regions (FR1, FR2, or FR3) of VH, and Thr5, Tyr50, Arg62, Ser64, Ser66, Ser68, Thr73, Thr75, Ser77, and Asp83 in the non-complementary determining regions (FR1, FR2, or FR3) of VL. The numbering of the above positions refers to SEQ ID NO:1 and 2.

In the preferred embodiment, the mutation positions in the heavy chain of the anti-CTLA-4 antibody Ipilimumab are selected from the group consisting of: Gln3, Arg19, Leu20, Ser25, Gly26, Phe27, Thr28, Phe29, Ser30, Ser31, Tyr32, Thr33, Met34, His35, Gly44, Phe50, Ile51, Ser52, Tyr53, Asp54, Gly55, Asn56, Asn57, Lys58, Tyr59, Tyr60, Ala61, Asp62, Ser63, Lys65, Gly66, Thr69, Ser71, Arg72, Asp73, Asn74, Ser75, Lys76, Asn77, Thr99, Gly100, Trp101, Leu102, Gly103, Pro104, Leu105, Asp106, and Tyr107; preferably selected from the group consisting of: Ser30, Ser31, Tyr32, Thr33, Ile51, Asp54, Gly55, Asn56, Lys58, Tyr59, Tyr60, Ala61, Asp62, Ser63, Lys65, Gly66, Gly100, Trp101, Leu102, Gly103, Pro104, Leu105, Asp106, and Tyr107.

In the preferred embodiment, the mutation positions in the light chain of the anti-CTLA-4 antibody Ipilimumab are selected from the group consisting of: Gln6, Arg24, Ala25, Ser26, Gln27, Ser28, Val29, Gly30, Ser31, Ser32, Tyr33, Leu34, Ala35, Tyr37, Ile49, Tyr50, Gly51, Ala52, Phe53, Ser54, Arg55, Ala56, Thr57, Gly58, Ile59, Pro60, Asp61, Arg62, Ser68, Gly69, Thr70, Gln90, Gln91, Tyr92, Gly93, Ser94, Ser95, Pro96, Trp97, Thr98, Phe99, and Gly100; preferably selected from the group consisting of: Ala25, Ser26, Ser28, Gly30, Ser31, Ser32, Leu34, Ala35, Gly51, Ala52, Ser54, Ala56, Thr57, Gly58, Ile59, Gly93, Ser94, Ser95, Thr98, and Gly100.

The above position numbers refer to SEQ ID NO:1 and 2.

It has been found (CN 201880072546.4) that after mutating the amino acid residues in the FR and CDR regions of the light chain variable region and heavy chain variable region of the anti-CTLA-4 antibody, the obtained mutant still maintains a relatively high binding affinity (more than 60% of the binding affinity of the wild-type antibody). All the contents about the CTLA-4 mutant disclosed herein are incorporated herein by reference.

Mutations, transfections, expressions, and purifications of biomolecules can be carried out by methods known in the field. For example, nucleic acids of anti-CTLA-4 antibodies with mutations at selected positions can be directly synthesized, and then nucleic acid molecules of different fragments obtained by enzymatic digestion can be ligated into an expression vector which can be transformed into bacterial or eukaryotic host cells. Biomolecules containing cysteine mutations can be obtained through recombination in host cells.

Bacterial or eukaryotic host cells applicable to the present invention can be commonly used host cells in the field, including but not limited to bacteria, yeast, and mammalian cells. Useful eukaryotic host cells include CHO cells, HEK293T cells, or *Pichia pastoris.*

Expression vectors applicable to the present invention can be virus-based expression vectors known in the field, including but not limited to baculovirus, simian virus (SV40), retrovirus, or vaccinia virus-based vectors. Expression vectors containing appropriate regulatory elements and selection markers can be used to prepare mammalian cell lines stably expressing mutants. For example, the GS eukaryotic expression system (Lonza), the DHFR eukaryotic expression system (Invitrogen), and the Pichia pastoris expression system (Invitrogen) can be used for expression and preparation.

The biomolecules of the present invention can be purified by separation methods known in the field. These methods include but are not limited to affinity chromatography, DEAE ion exchange column, gel filtration, and hydroxyapatite chromatography. For example, protein A column or G column can be used to separate antibody molecules in cell culture supernatant or cytoplasmic extract. In some embodiments, biomolecules can be "engineered" so that they contain amino acid sequences that allow the biomolecules to be captured onto an affinity matrix. For example, markers can be used to facilitate the purification of polypeptides. Suitable markers include but are not limited to c-myc, hemagonium, poly-His (e.g., 6His), and Flag TM (Kodak). Such markers can be inserted at any position within the polypeptide, including the carboxyl terminus or amino terminus. The biomolecule conjugates of the present invention can also be purified by immunoaffinity chromatography.

The functional group applicable to the present invention can be represented by the formula R1-R2-R3-L-R4. In the functional group, R1, R2, and R3 are linked together by any suitable linking method, including but not limited to amide bond, ester bond, carbamate bond, urea bond or hydrazone bond. R3 and R4 are linked by L, and L is a hydrazone bond. In the present invention, an amide bond can be represented by "-CO-NH-", an ester bond can be represented by "-C(O)O-", a carbamate bond can be represented by "-NH-C(O)-O-", a urea bond can be represented by "-NH-CO-NH-", and a hydrazone bond can be represented by "-C(R')=N-NH-", where R' is H or C1-4 alkyl. In this document, unless otherwise specified, R1, R2, R3, L, R4, and R5 are covalently linked to each other. In addition, it should be understood that when R1, R2, R3, L, R4, and R5 are part of a molecule, they refer to groups; when they are used as preparation raw materials, they refer to compound molecules. At this time, H or OH or other corresponding groups known in the field are usually linked at their linking positions (i.e., the wavy line positions shown below) to satisfy valence bond theory and can also react with other parts to form the connection bonds described herein, including but not limited to the amide bond, ester bond, carbamate bond, urea bond or hydrazone bond.

In this description, R1 is a protecting group for biomolecule R5. It can be selected from any group that can prevent the biomolecule from binding to its antigen, ligand or receptor, so as to prevent the biomolecule from being interfered by other molecules. For example, it prevents the biomolecule from binding to its ligand or receptor before the biomolecule reaches a pathological microenvironment such as a tumor or inflammatory microenvironment. Suitable R1 is selected from the group consisting of: NRₐR_{b}-R-CO-, C₁₋₄ alkoxy-(C₁₋₄ alkoxy)ₙ-carbonyl-C₁₋₄ alkylene-carbonyl-, 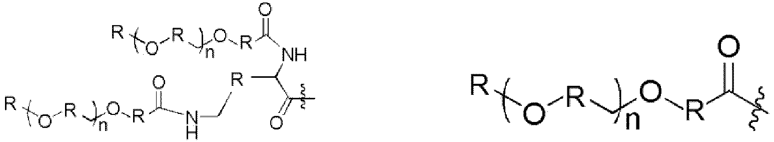 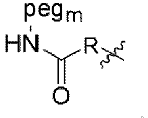 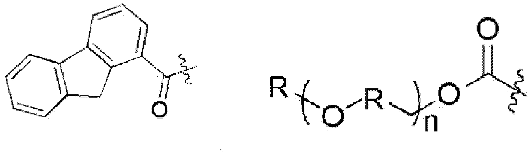 wherein each R is independently a C₁₋₄ alkyl group; each n is independently an integer within the range of 1 to 30000, for example 1-15000, 1-5000, 1-2000, 1-150, 1-50, 1-20 or 3-12; polyethylene glycol or pegₘ is polyethylene glycol with a molecular weight of 44 to 132000, for example polyethylene glycol with a molecular weight of 1000 to 50000 or 10000 to 30000; m represents the molecular weight of polyethylene glycol; the wavy line indicates the position where R1 is linked to R2; Rₐ is C₁₋₄ alkyl-O-[(CH₂)_{q}-O]ₙ-(CH₂)_{q}-; R_{b} is C₁₋₄ alkyl-O-[(CH₂)_{q}-O]ₙ-(CH₂)_{q}-C(O)-; q is an integer from 1 to 4. Preferably, the molecular weight of R1 does not exceed 20 kD.

In some embodiments, the NRₐR_{b}-R-CO- is: 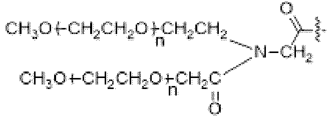 Preferably, each n is independently an integer from 1 to 1000.

In some embodiments, the C₁₋₄ alkoxy-(C₁₋₄ alkoxy)ₙ-carbonyl-C₁₋₄ alkylene-carbonyl- is: 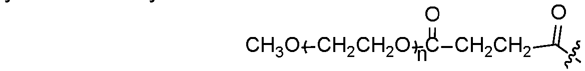 Preferably, each n is independently an integer from 1 to 1000.

In some embodiments, the R1 group is selected from the group consisting of:

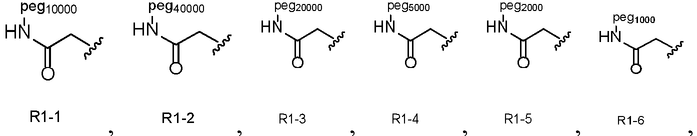

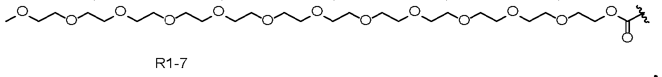

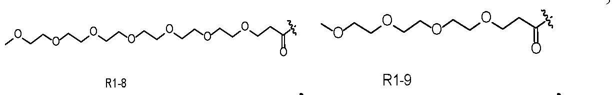

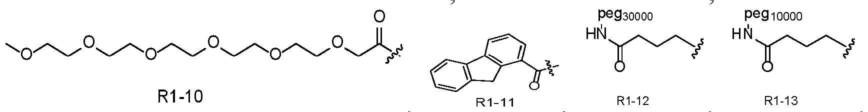

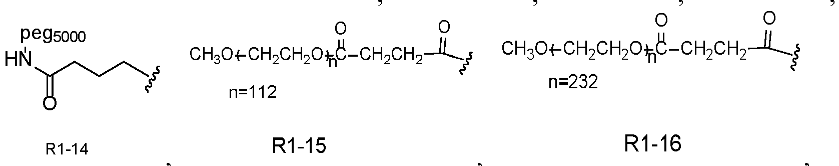

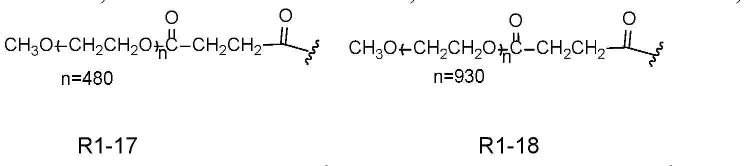

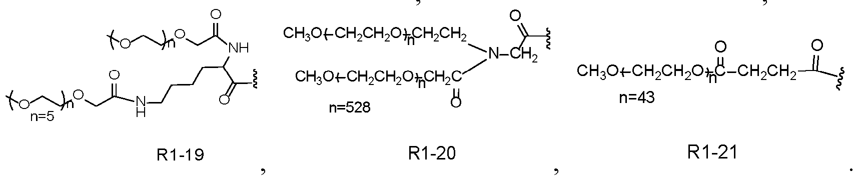

Generally, if the mutation position exists within the functional domains of the biomolecule, for example, in the CDR of an antibody, then there is no particular limitation on the molecular weight of R1, and R1 can have a relatively low molecular weight. If the mutation position is outside the functional domain of the biomolecule, for example, in the non-CDR of an antibody, it is preferable to select R1 so that the molecular weight of R1-R2-R3-L-R4 is higher than 200, preferably higher than 500, more preferably higher than 1000, so that the molecular weight of the biomolecule conjugate is 5000 or higher, preferably 8000 or higher, more preferably 10000 or higher, thereby better preventing the biomolecule from binding to its ligand or receptor before reaching the pathological microenvironment.

In the present invention, R2 is a linking arm that is hydrolyzed and activated by one or more proteolytic enzymes, proteases or peptidases in a pathological microenvironment. In the present invention, proteolytic enzymes, proteases or peptidases can be various proteolytic enzymes, proteases or peptidases existing in the pathological microenvironment. For example, proteases can be cysteine proteases, asparaginyl proteases, aspartic proteases, glutamic proteases, threonine proteases, gelatinases, metalloproteases or asparaginyl peptide cleavage enzymes; preferably, R2 is a peptide that can be cleaved by at least one selected from the following enzymes: asparaginyl endopeptidase, granzyme, cathepsin B, cathepsin C, cathepsin D, cathepsin E, cathepsin K, cathepsin L, kallikrein, hK1, hK10, hK15, plasmin, collagenase, type IV collagenase, astacin, factor Xa, chymotrypsin-like protease, trypsin-like protease, elastase-like protease, subtilisin-like protease, actinidin, bromelain, calpain, caspase, caspase-3, defensin cysteine protease, papain, HIV-1 protease, HSV protease, CMV protease, chymotrypsin, pepsin, lipase, plasmin, nepenthesin, metalloexopeptidase, metalloendopeptidase, matrix metalloproteinase (MMP), MMP1, MMP2, MMP3, MMP8, MMP9, MMP10, MMP11, MMP13, MMP14, ADAM10, ADAM12, urokinase plasminogen activator (uPA), neurokinin, prostate specific antigen (PSA, hK3), interleukin 113 converting enzyme, thrombin, FAP (FAP-a), transmembrane peptidase, dipeptidyl peptidase and dipeptidyl peptidase IV (DPPIV/CD26). In a preferred embodiment, the disclosure in the present invention particularly relates to Legumain, which is mainly expressed and secreted by tumor cells in the tumor microenvironment. Through the expression of Legumain, tumor-associated macrophages (M2 type) are also different from monocytes and inflammatory macrophages (M1 type). In the present invention, a polypeptide is a substrate of a proteolytic enzyme and can be recognized and cleaved by a proteolytic enzyme.

The R2 group of the present invention can be represented by -R2a-, -R2b-, -R2a-N-, -R2a-D-, -R2a-AAN-, -R2a-AAD-, or -R2a-R2b-; wherein R2a is a peptide that can be cleaved at the amide bond by one or more proteolytic enzymes; R2b is a peptide that can form a carbamate with R3 through the amino group in the side chain, and the carbamate can be cleaved by one or more proteolytic enzymes; A is alanine; N is asparagine, the amino group in its side chain forms a carbamate with R3, and the carbamate can be cleaved by Legumain; D is aspartic acid, the amino group in its side chain forms a carbamate with R3 and can be cleaved by granzyme B. R2a and R2b can be linked by forming an amide bond. After Legumain and granzyme B cleave the bond (such as a carbamate) between R2 and R3, under acidic conditions, L in R3-L- is cleaved, thereby releasing the antibody with the R4 group. In some embodiments, a suitable polypeptide that can be activated by proteolytic enzymes can be a tripeptide. Any substrate peptide that can be recognized and cleaved (activated) by proteolytic enzymes in a pathological microenvironment known in the field can be used as R2 disclosed herein. Such peptides have the structure disclosed in WO 2016/026458, and the entire content thereof is incorporated into this disclosure by reference. In some embodiments, in the tripeptide structure suitable for the present invention, the amino acid residue linked to R1 can be selected from Ala, Thr, Val and Ile; the intermediate amino acid residue can be selected from Ala, Thr, Val and Asn; and the amino acid residue linked to R3 can be selected from Asn and Asp. Generally, R2 is linked to R1 through the amino group of its amino acid residue by an amide bond, ester bond, carbamate bond, urea bond or hydrazone bond, and is linked to R3 through the carboxyl group of its amino acid residue by an amide bond, ester bond, carbamate bond, urea bond or hydrazone bond. In some preferred embodiments of the present invention, R2 is a tripeptide selected from Ala-Ala-Asn and Ala-Ala-Asp. Ala-Ala-Asn can be recognized and cleaved by Legumain, and Ala-Ala-Asp can be recognized and cleaved by granzyme.

In the present invention, the R3 group is a linking arm, including but not limited to -HN-phenyl-R'-, wherein R' is a bond. Preferably, the NH group and R' are located at the para position of the phenyl group. When preparing the compound of the present invention, compounds represented by the following formulas R3-1 and R3-2 can be used to prepare the R3 linking arm of the present invention:

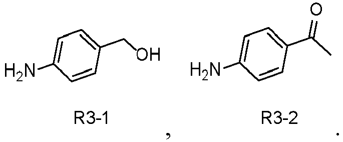

In some embodiments, the L group is a chemical bond that can be activated by acidity in a pathological microenvironment, specifically a hydrazone bond (i.e., -C(R')=N-NH-, where R' is H or C₁₋₄ alkyl). For example, in some embodiments, the structure of R1-R2-R3-L-R4 can be represented as follows:

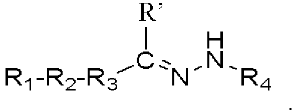

It should be understood that the L group can be obtained through a chemical reaction between the R3 compound and the R4 compound.

In the present invention, the R4 group is a binding group that, after the cleavage of the R2 group and the R3 group, can restore, maintain, reduce, or promote the binding ability of the anti-CTLA-4 antibody with its antigen, ligand, or receptor. In some embodiments, after the cleavage of the R2 group and the R3 group, the resulting R4-s-Cys-R5 exhibits >60% affinity for the antigen, ligand or receptor of native R5.

A suitable R4 group can be represented by the following formula: 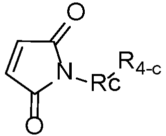 wherein, Rc is selected from C1-12 alkylene, C1-12 alkylene-O-C1-12 alkylene, C1-12 alkylene-C3-8 cycloalkyl, (C1-4 alkylene-O)p-C1-12 alkylene, C1-12 alkylene-carbonyl amino-(C1-4 alkylene-O)p-C1-12 alkylene, -phenyl-C1-12 alkylene, C3-8 cycloalkyl, C1-12 alkylene-C3-8 cycloalkyl-C1-12 alkylene, C1-12 alkylene-NHCO-O-C1-12 alkylene, C1-12 alkylene-COO- and C1-12 alkylene-phenyl-C1-12 alkylene; p is an integer from 1 to 10, preferably 1, 2, 3 or 4;
R_{4-c} is a bond or -CO-;
wherein, the L group is linked to the R4 group through R_{4-c} of the R4 group.

Generally, the R4 group is linked to the S atom of cysteine of R5 through maleimide.

In the preferred embodiment, Rc is C1-12 alkylene and R_{4-c} is -CO-; wherein the R4 group is linked to the L group through its carbonyl group.

In some embodiments, the R4 group can be prepared from R4 compounds selected from the group consisting of:

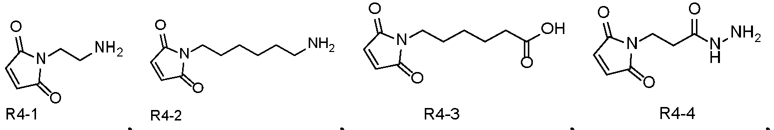

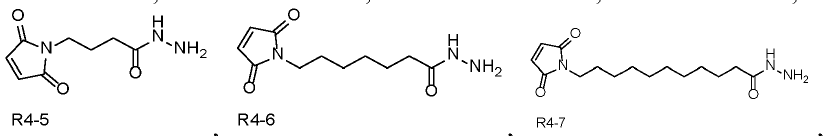

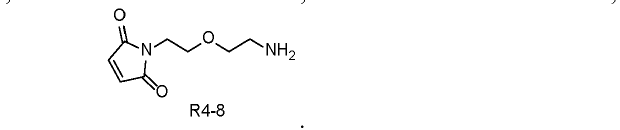

It should be understood that in the present invention, the wavy line used in each indicated formula indicates the connection position of the part containing the wavy line with other parts.

As mentioned above, R5 represents an anti-CTLA4 antibody in which one or more amino acid residues are mutated to cysteine. R5 is actually a biomolecule in which the sulfhydryl group of the introduced cysteine lacks a hydrogen atom. The lack of this sulfhydryl hydrogen atom allows R5 to be regarded as a group linked to R4 of the present invention.

In one or more embodiments of the present invention, the anti-CTLA-4 antibody can be activated in multiple ways. One way is to cut off R1-R2 from R3-L-R4-S-cys-R5 by proteolytic enzymes. Another method is to cleave R1-R3-L in the pathological microenvironment under acidic conditions to release R4-S-cys-R5. The present invention has found that the dual-activated (enzyme-activated and acid-activated) compounds of the present invention have higher activation efficiency in diseased tissues compared to single-activated compounds.

The conjugate of the present invention can be prepared by methods including reducing the mutated biomolecule with DTT, TCEP or other reducing agents; oxidize with Cu₂SO₄, dehydroascorbic acid or other oxidants; then, under liquid-phase or solid-phase conditions, combine the oxidized biomolecule (R5) with R1-R2-R3-L-R4. The final product can be collected in the liquid phase.

Therefore, in addition to the conjugate, the present invention also contains functional moieties, that is R1-R2-R3-L-R4; R2-R3-L-R4; R3-L-R4-S-cys-R5; R4-S-cys-R5 and the mutated biomolecule; wherein R1, R2, R3, L, R4, R5 and their linking methods and the mutated biomolecule are as defined in any part or any embodiment of the present invention.

In some embodiments, the functional moieties are shown by CP3, CP7, CP13, CP23-CP27. In the present invention, -S-cys- indicates that R4 is covalently linked to R5 via the sulfhydryl group of the cysteine introduced by the mutation in R5. R4-S-cys-R5 is a conjugate formed under acidic conditions in the pathological microenvironment.

The conjugate, functional moiety, R2-R3-L-R4, R3-L-R4-S-cys-R5 and R4-S-cys-R5 as described herein can be synthesized by methods known in the art. For example, they can be prepared according to the methods described in Example 1 of this application.

The present invention also includes a pharmaceutical composition comprising the conjugate as described herein. The pharmaceutical composition may further comprise a pharmaceutically acceptable carrier. The carrier can be any pharmaceutically acceptable carrier or excipient, which can vary according to the dosage form and administration route. Pharmaceutically acceptable carriers are usually safe and non-toxic, and can contain any known substance used in formulating pharmaceutical compositions in the pharmaceutical industry, including fillers, diluents, coagulants, binders, lubricants, glidants, stabilizers, colorants, wetting agents, and disintegrants, etc. Suitable pharmaceutically acceptable carriers include sugars such as lactose or sucrose, mannitol or sorbitol; cellulose preparations and/or calcium phosphates such as tricalcium phosphate or calcium hydrogen phosphate; starches including corn starch, wheat starch, rice starch, potato starch, gelatin, tragacanth gum, methylcellulose, hydroxypropyl methylcellulose, sodium carboxymethylcellulose and/or polyvinylpyrrolidone; silica, talc, stearic acid or its salts such as magnesium stearate or calcium stearate; and/or polyethylene glycol and so on. When choosing a pharmaceutically acceptable carrier, the main consideration is the administration route of the drug dosage form. This is well known in the field.

A pharmaceutical composition may comprise a therapeutically or prophylactically effective amount of a conjugate. "Effective amount" indicates an amount of a component sufficient to produce a desired response. The specific effective amount depends on various factors such as the specific disease to be treated, the physical condition of the patient, such as weight, age and gender, the duration of treatment, co-administered therapies (if any), and the specific formulation used. Generally, a "effective amount" as described herein is a conventional amount of a biomolecule. However, in some embodiments, the therapeutically or prophylactically effective amount of the conjugate contained in the pharmaceutical composition of the present invention may be lower than the conventional amount of biomolecules but can produce better therapeutic or prophylactic effects because the biomolecules are protected by protecting groups before reaching the pathological microenvironment and binding to their ligands or receptors.

The pharmaceutical composition of the present invention can be formulated into various suitable dosage forms, including but not limited to tablets, capsules, injections, etc., and can be administered by any suitable route to achieve the intended purpose. For example, it can be administered parenterally, subcutaneously, intravenously, intramuscularly, intraperitoneally, transdermally, orally, intrathecally, intracranially, intranasally or externally. The dose of the drug can depend on the patient's age, health status and weight, concurrent treatments, and the frequency of treatment. The pharmaceutical composition of the present invention can be administered to any subject in need thereof, such as a mammal, especially a human being.

In tumor patients, tumor cells or antigen-presenting cells (APCs) carrying tumor antigens partially or completely inhibit the host's immune killing of tumors by binding to T cells. However, the conjugate of the present invention is activated and released in the pathological microenvironment by proteolytic enzymes, especially Legumain or granzyme, and/or under acidic conditions.

Therefore, the conjugate of the present invention can effectively break through the individual's immune barrier and reach the pathological microenvironment, and then be activated and released in the pathological microenvironment. As a result, it can selectively promote the proliferation or cytotoxic effects of T cells in tumor or inflammatory microenvironments, thereby achieving low autoimmunity and high potency.

Therefore, each conjugate or biomolecule of R4-S-cys-R5 or mutated biomolecule disclosed in this invention can be used for the treatment of tumors or inflammation, or can be used as the active ingredient for preparing the medicine for the treatment of tumors or inflammation. The tumor or inflammation described herein is preferably CTAL-4-mediated tumor or inflammation, and can be any tumor or inflammation that can be treated by anti-CTAL4 antibody (especially the anti-CTLA4 antibody described herein, such as Ipilimumab), including but not limited to melanoma, bladder, brain, breast, cervix, colon-rectum, esophagus, kidney, liver, lung, nasopharynx, pancreas, prostate, skin, stomach, uterus, ovary, testis and cancers in the blood, etc. Specifically, cancers include bladder cancer, brain cancer, breast cancer, cervical cancer, colorectal cancer, esophageal cancer, kidney cancer, liver cancer, lung cancer, nasopharyngeal cancer, pancreatic cancer, prostate cancer, skin cancer, stomach cancer, uterine cancer, ovarian cancer, testicular cancer and blood cancer.

Also included are methods for treating or preventing tumors or inflammations, which comprise administering to a subject in need thereof a therapeutically or prophylactically effective amount of a conjugate as described herein or a pharmaceutical composition thereof. The method can be used in combination with any known radiotherapy or immunotherapy.

It should be understood that the terms "comprise" and "include" or similar expressions used in the present invention also mean "consist of" and the like. The sum of all weight percentages or volume percentages should be equal to 100%. Unless otherwise stated, various reagents and products used in the examples are commercial products. Unless otherwise stated, the methods mentioned in the examples are implemented according to conventional techniques. The following examples are not intended to limit the scope of the present invention.

Example 1: Synthesis of functional groups CP1-CP30.

When R1, R2, R3, L and R4 are selected from different groups, the synthesized compounds are shown in Table 1-1 and Table 1-2 below.

**Table 1-1: Compound names and R1, R2, R3 and R4 compounds used to form the compounds**

| Name | R1 | R2 | R3 | R4 |
|---|---|---|---|---|
| CP1 | R1-7 | AAN | R3-1 | R4-1 |
| CP2 | R1-7 | AAD | R3-1 | R4-1 |
| CP3 | R1-7 | AAN | R3-1 | R4-4 |
| CP4 | R1-7 | AAD | R3-1 | R4-3 |
| CP5 | R1-7 | AAN | R3-1 | R4-2 |
| CP6 | R1-7 | AAD | R3-1 | R4-2 |
| CP7 | R1-7 | AAD | R3-2 | R4-4 |
| CP8 | R1-10 | / | R3-2 | R4-4 |
| CP9 | R1-12 | / | R3-1 | R4-4 |
| CP10 | R1-13 | / | R3-1 | R4-4 |
| CP11 | R1-13 | / | R3-2 | R4-4 |
| CP12 | R1-7 | AAN | R3-1 | R4-4 |
| CP13 | R1-7 | AAN | R3-1 | R4-4 |
| CP14 | R1-10 | / | R3-1 | R4-4 |
| CP15 | R1-11 | AAN | R3-1 | R4-3 |
| CP16 | R1-9 | / | / | R4-3 |
| CP17 | R1-8 | / | / | R4-3 |
| CP18 | R1-15 | / | / | R4-3 |
| CP19 | R1-12 | / | R3-2 | R4-4 |
| CP20 | R1-15 | AAD | R3-1 | R4-2 |
| CP21 | R1-16 | AAN | R3-1 | R4-2 |
| CP22 | R1-16 | AAD | R3-1 | R4-2 |
| CP23 | R1-15 | AAN | R3-1 | R4-4 |
| CP24 | R1-16 | AAN | R3-1 | R4-4 |
| CP25 | R1-17 | AAN | R3-1 | R4-4 |
| CP26 | R1-20 | AAN | R3-1 | R4-4 |
| CP27 | R1-21 | AAN | R3-1 | R4-4 |
| CP28 | R1-15 | AAN | R3-1 | R4-2 |
| CP29 | R1-21 | AAN | R3-1 | R4-2 |
| CP30 | R1-20 | AAN | R3-1 | R4-2 |
| CP31 | / | / | R3-1 | R4-4 |

**Table 1-2: Compound structures**

| Name | Chemical formula |
|---|---|
| CP1 | 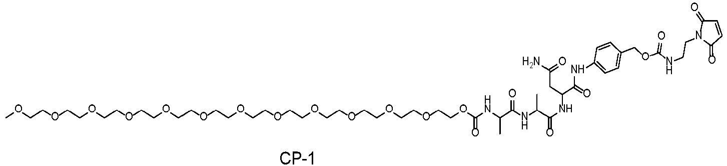 |
| CP2 | 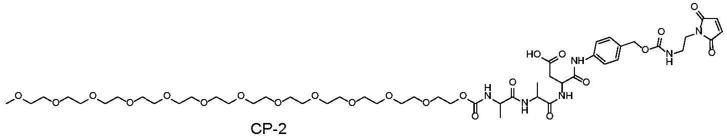 |
| CP3 | 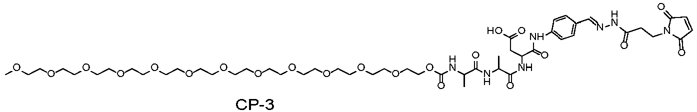 |
| CP4 | 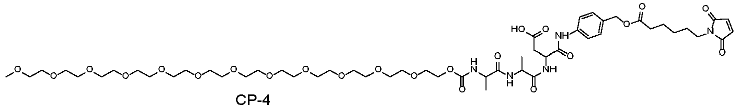 |
| CP5 | 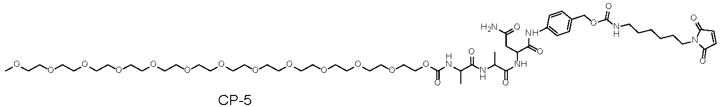 |
| CP6 | 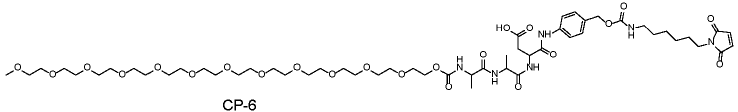 |
| CP7 | 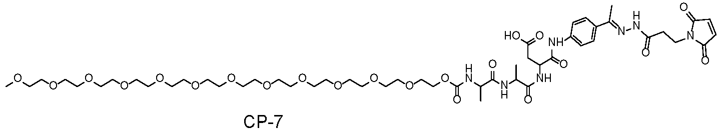 |
| CP8 | 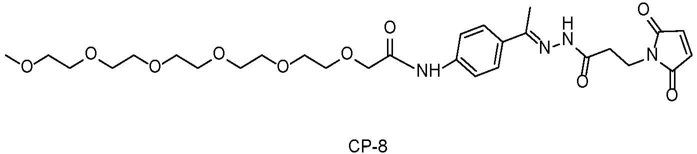 |
| CP9 | 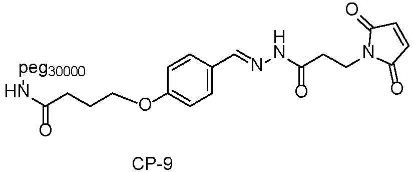 |
| CP10 | 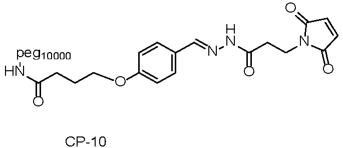 |
| CP11 | 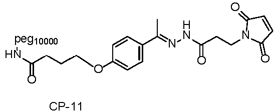 |
| CP12 | 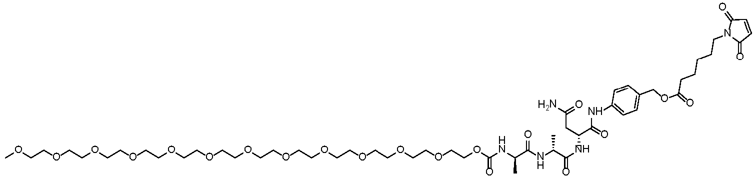 |
| CP13 | 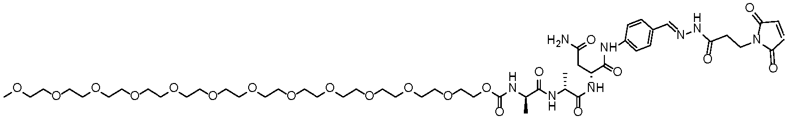 |
| CP14 | 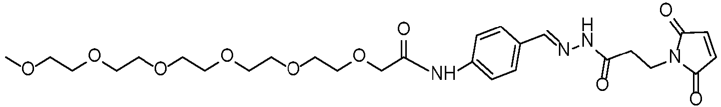 |
| CP15 | 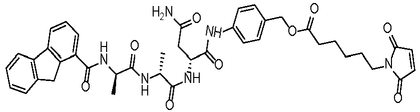 |
| CP16 | 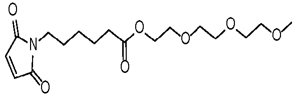 |
| CP17 | 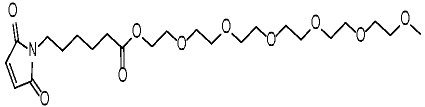 |
| CP18 | 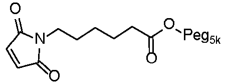 |
| CP19 | 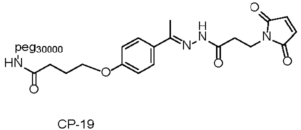 |
| CP20 | 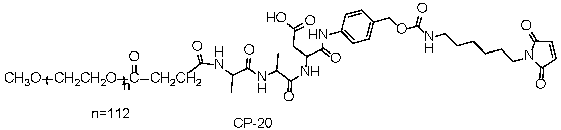 |
| CP21 | 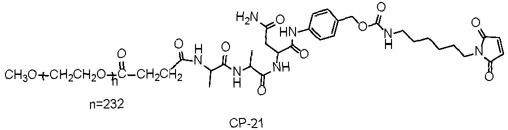 |
| CP22 | 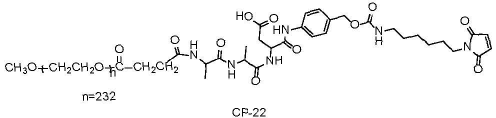 |
| CP23 | 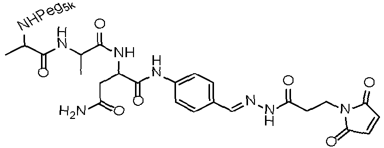 |
| CP24 | 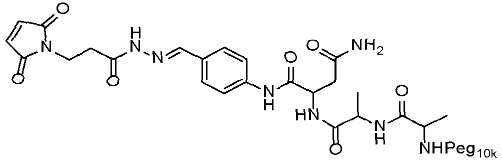 |
| CP25 | 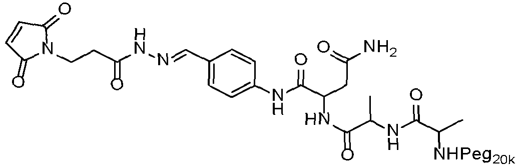 |
| CP26 | 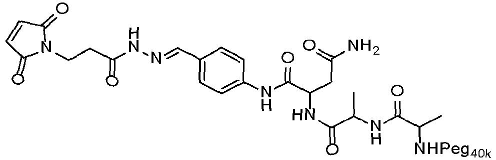 |
| CP27 | 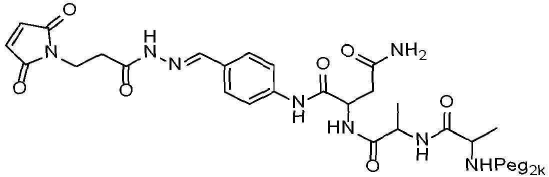 |
| CP28 | 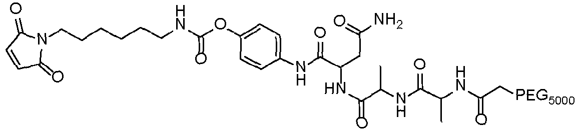 |
| CP29 | 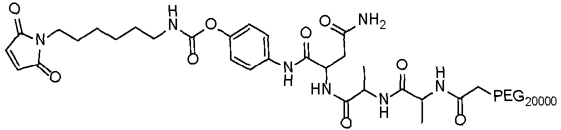 |
| CP30 | 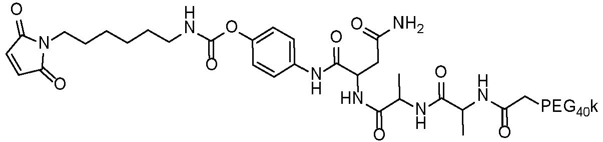 |
| CP31 | 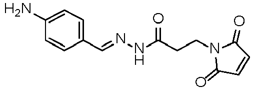 |

When R2 is Ala-Ala-Asn and R3 is PABC (R3-1), the synthesis route is shown as follows:

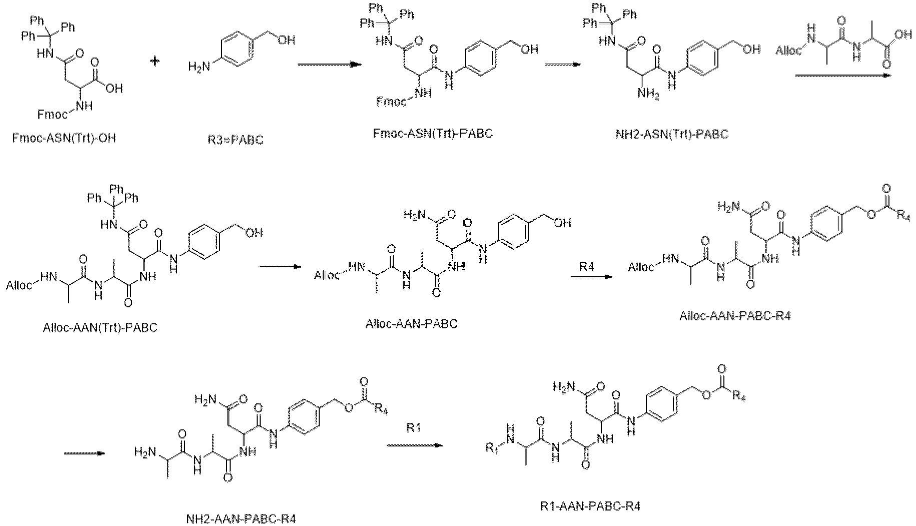

Taking CP5 as an example, the synthesis route is shown as follows: 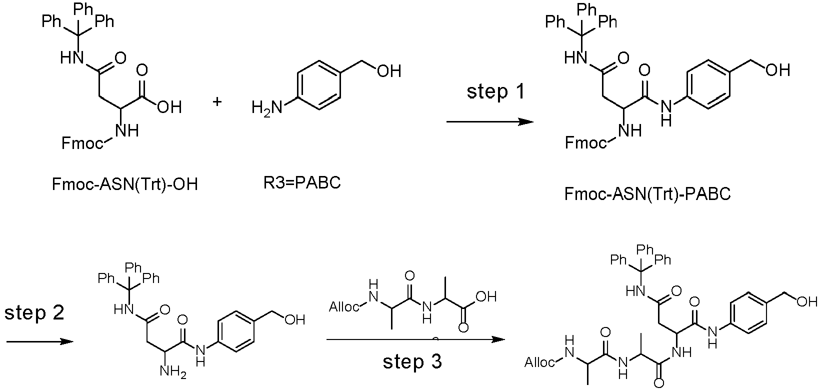 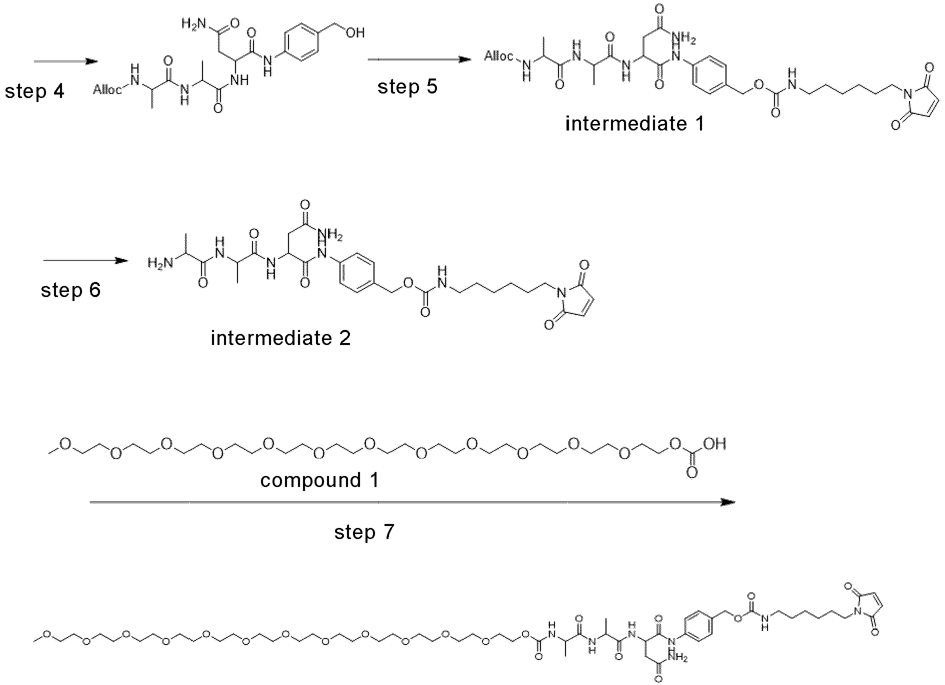
1) Fmoc-Asn(Trt)-OH (20 g, 0.03 mol),
   2-(7-azabenzotriazole)-N,N,N',N'-tetramethyluronium hexafluorophosphate (HATU) (15 g, 0.04 mol) and DMF (200 mL) were added to a three-necked flask and stirred for 30 minutes. P-aminobenzyl alcohol (4.1 g, 0.03 mol) and N,N-diisopropylethylamine (8.7 g, 0.06 mol) were added respectively at 0 °C, and then stirred at room temperature for 3 hours. Most of the DMF was removed by rotary evaporation. The residue was dissolved in ethyl acetate (200 mL), washed successively with saturated ammonium chloride solution and saturated sodium chloride solution, dried over anhydrous sodium sulfate, and then filtered. Evaporating off the solvent and trituration the crude product to obtain a white solid Fmoc-Asn (Trt)-PABC (21.3 g; yield: 90%).
2) Fmoc-Asn (Trt)-PABC (16.0 g, 22 mmol) was dissolved in N, N-dimethylformamide (80 mL). Piperidine was added (30 mL), and then the above solution was stirred at room temperature for 2 hours. The solvent was removed by evaporation under reduced pressure. The residue was dried under high vacuum in a vacuum oven to remove a small amount of piperidine to obtain 9.8 g of a pale yellow solid NH2-Ann (Trt)-PABC, which can be used in the next step without purification.
3) Alloc-Ala-Ala-OH (5.0 g, 20.4 mmol), benzotriazole-N,N,N',N'-tetramethyluronium hexafluorophosphate (HBTU) (11.6 g, 30.6 mmol) and DMF (50 mL) were added into a three-necked flask and stirred for 30 minutes in an ice bath. NH2-Asn (Trt)-PABC (9.8 g, 20.4 mmol) and N,N-diisopropylethylamine (7.89 g, 61.2 mmol) were added into the above three-necked flask at 0 °C respectively, and then stirred overnight at room temperature. The solvent removed by evaporation under reduced pressure. The residue was dissolved in ethyl acetate (200 mL), washed successively with saturated ammonium chloride solution and saturated sodium chloride solution, dried with anhydrous sodium sulfate and filtered. Evaporating to remove the solvent. The obtained crude product was recrystallized to obtain a white solid Alloc-AAN(Trt)-PABC (13.0 g; yield: 90%).
4) Alloc-AAN(Trt)-PABC (10.0 g, 14.2 mmol) was dissolved in dichloromethane (100 ± 15 mL). Trifluoroacetic acid (20 mL) was added, and then stirred at room temperature for 4 hours. After washing and fractionation with water, the organic phase was dried over anhydrous sodium sulfate. The solvent was removed by evaporation under reduced pressure, and the residual trifluoroacetic acid was removed by high vacuum evaporation. The crude product was separated by column chromatography to obtain Alloc-AAN-PABC (5.9 g; yield: 89%).
5) Alloc-AAN-PABC (467 mg, 1.01 mmol) dissolved in dichloromethane (10 mL) was added to a three-necked flask. Under ice bath and nitrogen protection, 4-nitrophenyl chloroformate (406 mg, 2.02 mmol) in dichloromethane and pyridine (160 mg, 2.03 mmol) in dichloromethane were added dropwise to the flask respectively, and stirred overnight at room temperature. 1-(6-aminohexyl)-1H-pyrrolo [2,5]-dione (235 mg, 1.2 mmol) was added in batches to the above solution and reacted at room temperature for 4 hours. The reaction solution was dried by rotary evaporation. The obtained crude product was purified by silica gel column chromatography to obtain a white solid S15-1 (540 mg; yield: 80%).
6) DMF (10 ml), S15-1 (208 mg, 0.31 mmol), acetic acid (274 mg, 4.65 mmol), triphenylphosphine (72 mg, 0.062 mmol) and tributyltin hydride (1.17 g, 4.03 mmol) were added successively to a single-necked flask. After replacing the air in the flask with nitrogen, the mixture was stirred at room temperature until S15-1 is completely reacted. After the reaction is completed, DMF was removed by evaporation under reduced pressure. The crude product was separated and purified by silica gel column chromatography to obtain S15-2 (white solid, 116 mg, yield: 62%).
7) Compound 1 (940 mg, 0.18 mmol), benzotriazole-N,N,N',N'-tetramethyluronium hexafluorophosphate (HBTU) (95 mg, 0.25 mmol) and DMF (10 mL) were added to a three-necked flask, and then stirred for 30 minutes in an ice bath. Then compound intermediate 2 (108 mg, 0.18 mmol) and N,N-diisopropylethylamine (70 mg, 0.54 mmol) were added respectively at 0 °C, and then stirred overnight at room temperature. The solvent was removed by evaporation under reduced pressure. The crude product was separated and purified by silica gel column chromatography to obtain a white solid (85.5 mg; yield: 40%), which is compound CP5.

When R2 has the amino acid sequence of Ala-Ala-Asp and R3 is PABC, the synthesis route is shown as follows:

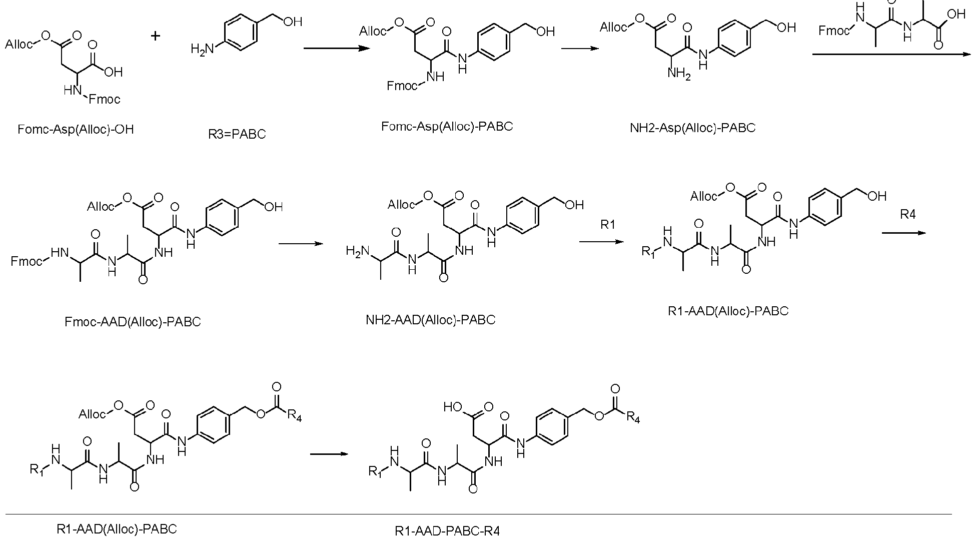

CP1, CP2, CP4, CP6, CP12, CP13, CP15, CP20-CP22, and CP28-CP30 were synthesized using the above method.

When containing an acid-activatable chemical bond, the synthesis scheme is shown as follows:

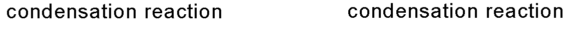

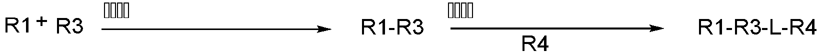

As shown in CP8, the specific synthesis process is as follows: 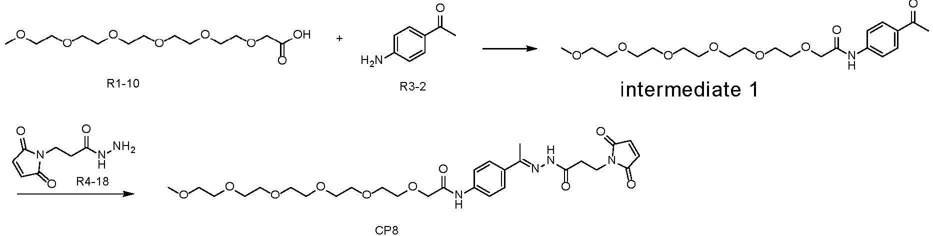
1) R1-10 (3.1 g, 0.01 mol), 2-(7-azabenzotriazole)-N,N,N',N'-tetramethyluronium hexafluorophosphate (HATU) (4.56 g, 0.012 mol) and DMF (20 mL) were added to a three-necked flask and stirred for 30 minutes. R3-2 (1.35 g, 0.01 mol) and N,N-diisopropylethylamine (3.87 g, 0.03 mol) were added respectively at 0 °C, and then stirred at room temperature for 3 hours. DMF was removed by rotary evaporation. The residue was refined by silica gel column chromatography to obtain an intermediate 1 (2.5 g; yield 59%) as a light yellow oily substance.
2) Intermediate 1 (98 mg, 0.23 mmol) and R4-18 (42 mg, 0.23 mmol) were weighed in sequence and added to a 50 ml single-necked flask. Dichloromethane (5 mL) was added to dissolve intermediate 1 and R4-18, and then 4A molecular sieve (81 mg) was added. After replacing the air in the flask with nitrogen, the mixture was allowed to react overnight at room temperature. The reaction solution was dried by rotary evaporation. The crude product was purified by silica gel column chromatography to obtain white solid (81 mg; yield: 60%), which is compound CP8.

CP7, CP9-11, CP14 and CP19 were synthesized by the above method.

Taking CP23 as an example, when the amino acid sequence of R2 is Ala-Ala-Asn and L is an acid-activatable chemical bond, the synthesis scheme is as follows:

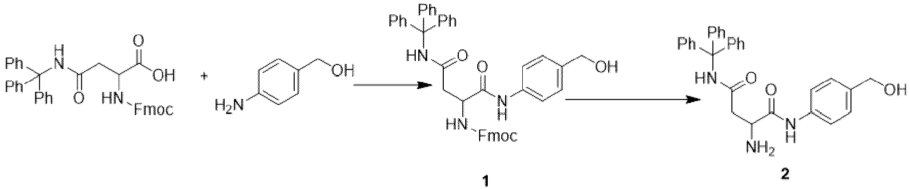

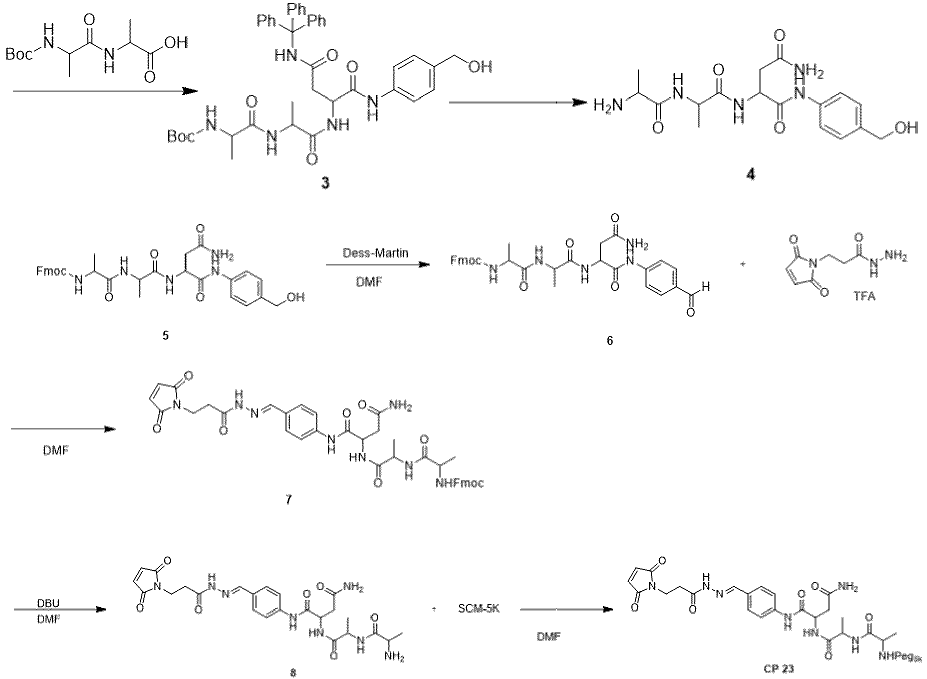

### 1) Synthesis of intermediate 1

500 ml of THF was added to a dry and clean 2 L reaction flask, 80 g of Fmoc-Asn(Trt)-OH was add to the reaction flask, stirred to dissolve, 46.6 g of DEPBT was added and stirred at room temperature for 15 minutes, 16 g of PABC was added. After reacting at room temperature for 30 minutes, 45 ml of DIPEA was added. After replacing the air in the flask with nitrogen, the mixture was stirred at room temperature for 3 hours. The reaction was monitored by TLC until the reaction was completed (Fmoc-Asn(Trt)-OH completely reacted). The reaction solution was evaporated under reduced pressure. A small amount of DMF (180 ml) was added to dissolve. The solution was added dropwise into 3 L of stirred water to precipitate a light yellow solid. After washing 2-3 times with water, filtering under suction, the solid was collected and dried in vacuum to obtain an off-white solid (yield above 90%).

### 2) Synthesis of intermediate 2.

500 ml of THF and the off-white solid obtained in the previous step were added sequentially to a 2 L single-necked reaction flask. Stirred to dissolve. Cooled down to 0 - 5 °C with an ice-salt bath. 100 ml of piperidine was added dropwise. After the dropwise addition is completed, gradually returned to room temperature and reacted for 1 hour. Monitor the reaction by TLC until it is completed. Evaporating the solvent under reduced pressure. A small amount of DMF was added to dissolve and dropwise into 2 L of stirred water. Mechanically stirred for 30 minutes. Filtered under suction. Repeat washing with water 2 - 3 times. Filtering under suction. 800 ml of methyl tert-butyl ether was added to the filter cake. Stirred for 30 minutes. Filtered under suction. Washing the filter cake twice with PE:EA = 10:1. Filtering under suction. The filter cake was collected. After vacuum drying, 80 g of an off-white solid with a purity of 70% was obtained.

### 3) Synthesis of intermediate 3.

50 ml of THF, 5.04 g of Boc-Ala-Ala-OH, and 3.89 g of DEPBT were added in sequence in a dry and clean 250 ml single-necked reaction flask. Reacting at room temperature for 10 minutes. 2.6 g of NH2-Asn(Trt)-PABC was added. After replacing the air in the flask with nitrogen, the mixture was stirred at room temperature for 15 minutes. 3.5 ml of DIPEA was added dropwise. And after replacing the air in the flask with nitrogen, the mixture was stirred at room temperature for 3 hours. Evaporating the solvent under reduced pressure. Trituration was performed with water 2 - 3 times. Filtering under suction to obtain a light yellow solid, 3.7 g. The solid was purified by column chromatography to obtain 2.0 g of product with a purity of 94.8% and a yield of 26.6%.

### 4) Synthesis of intermediate 4.

1.8 g of Boc-Ala-Ala-Asn(Trt)-PABC was added to a 100 ml single-necked reaction flask. 28.5 ml of TFA was added and 1.5 ml of water was added dropwise. Reacting at room temperature for 30 minutes. Monitoring the reaction by TLC until it is completed. Evaporating the solvent under reduced pressure. Trituration was performed with methyl tert-butyl ether. Filtering under suction to obtain a solid. The solid was dissolved in a dioxane:water = 1:1 solution. Adjusting the pH to 13 with 1N sodium hydroxide. Stirring at room temperature for 40 minutes. Evaporating the solvent under reduced pressure. Mixing with silica gel and purifying by column chromatography to obtain 450 mg of product. Yield: 47.5%.

### 5) Synthesis of intermediate 5.

100 ml of dioxane, 9.8 g of NH2-AAN-PABC were added to a 500 ml three-necked reaction flask in sequence. Then 3.28 g of Na₂CO₃ (prepared as a saturated aqueous solution and added dropwise) was added. The above mixture was cooled down to 0 - 5 °C with an ice-salt bath. 16.6 g of Fmoc-C was dissolved in 10 ml of dioxane and dropped into the above reaction solution. Gradually heating up to room temperature and reacting for 30 minutes (a solid precipitated and THF was added to continue the reaction). When the reaction was terminated, the solvent was evaporated under reduced pressure. Trituration was performed with water. Filtering under suction to obtain an off-white solid. Trituration was performed with methyl tert-butyl ether. Filtering under suction to obtain an off-white solid. The solid was dried in vacuum to constant weight to obtain 15.5 g of product with a purity of 80% and a yield of 80%.

### 6) Synthesis of intermediate 6.

Intermediate 5 (5 g, 8.3 mol) was dissolved in DMF (40 ml). Under stirring at room temperature, Dess-Martin reagent (5.3 g, 12.5 mmol) was added and continue stirring for 2 hours. The reaction solution was quenched successively with saturated Na₂S₂O₄ solution and saturated NaHCO₃ solution. Filtering. The filtrate was concentrated under reduced pressure. Then the solid was washed with water to obtain intermediate 6 (4.7 g of brown solid, yield: 94%).

### 7) Synthesis of intermediate 7.

Intermediate 6 (566 mg, 0.94 mmol) was dissolved in DMF (10 ml). Under stirring, compound 2 (280 mg, 0.94 mmol) was added. Under nitrogen protection, stirring overnight at room temperature. The reaction solution was prepared by medium pressure to obtain intermediate 7 (719 mg of white solid, yield: 100%).

### 8) Synthesis of intermediate 8.

intermediate 7 (280 mg, 0.37 mmol) was dissolved in DMF (10 ml). Under stirring, DBU (5 ul) was added. Stirring at room temperature for 3 hours. Then DBU (5 ul) was added again and continue stirring for 2 hours. The reaction solution was prepared by high pressure to obtain intermediate 8 (85 mg of white solid, yield: 42%).

### 9) Synthesis of final product CP23.

The compound SCM-5K (1.45 g, 0.29 mmol) was dissolved in DMF (20 ml). Under stirring, intermediate 8 (190 mg, 0.35 mmol) was added. Stirring overnight at room temperature. The reaction solution was concentrated under reduced pressure. Purifying by high-performance liquid chromatography to obtain product CP23 (1.12 g of white solid, yield: 77%).

CP3, CP5, CP16-CP18, and CP24-CP27 were synthesized by the above method.

The compounds CP1-CP30 were verified by mass spectrometry (MS). Their molecular weights are shown in Table 2, which are consistent with the molecular weights calculated based on their structures.

**Table 2: Mass spectrometry information table of functional groups of the present invention and its properties and yields.**

| Number | MS | Molecular Weight | Property | Yield |
|---|---|---|---|---|
| CP1 | 1132.23 | 1132 | white solid | 71mg |
| CP2 | 1133.21 | 1133 | white solid | 49mg |
| CP3 | 1130.21 | 1130 | white solid | 236mg |
| CP4 | 1160.28 | 1160 | white solid | 93mq |
| CP5 | 1188.33 | 1188 | white solid | 85.5mg |
| CP6 | 1189.32 | 1189 | white solid | 46mg |
| CP7 | 1144.24 | 1144 | white solid | 158mg |
| CP8 | 592.65 | 593 | white solid | 81mg |
| CP9 | 30354.38 | 30354 | white solid | 1534mg |
| CP10 | 10354.38 | 10354 | white solid | 728mg |
| CP11 | 10368.38 | 10368 | white solid | 1258mg |
| CP12 | 1159.29 | 1159 | white solid | 31mg |
| CP13 | 1129.23 | 1129 | white solid | 64mg |
| CP14 | 578.62 | 579 | white solid | 70mq |
| CP15 | 764.84 | 765 | white solid | 38mg |
| CP16 | 357.4 | 357 | white solid | 54mq |
| CP17 | 489.56 | 490 | white solid | 89mg |
| CP18 | 5139.22 | 5139 | white solid | 167mg |
| CP19 | 30368.41 | 30368 | white solid | 1002mg |
| CP20 | 5644.64 | 5645 | white solid | 81mg |
| CP21 | 10923.64 | 10924 | white solid | 106mg |
| CP22 | 10924.64 | 10925 | white solid | 97mg |
| CP23 | 5541.55 | 5542 | white solid | 1120mg |
| CP24 | 10541.55 | 10542 | white solid | 876mg |
| CP25 | 20541.55 | 20542 | white solid | 143mq |
| CP26 | 40541.25 | 40541 | white solid | 1025mg |
| CP27 | 2541.55 | 2542 | white solid | 136mg |
| CP28 | 5628.67 | 5629 | white solid | 121mg |
| CP29 | 20628.67 | 20629 | white solid | 2023mg |
| CP30 | 40628.67 | 40629 | white solid | 1184mg |
| CP31 | 286.29 | 286 | white solid | 30mg |

### Example 2: Construction of a series of anti-hCTLA4 TMEAbody with different blocking groups and linkers.

A series of anti-human CTLA4-TMEAbody (TMEAbody is the abbreviation of tumor microenvironment activated antibody) are constructed by site-specific coupling of different compounds (including blocking groups and linkers) with Ipilimumab containing Y49C mutation on the light chain (Y49C is according to the Kabat numbering system of antibodies, and this number corresponds to Y50C if numbered sequentially). The Ipilimumab antibody containing Y49C (abbreviated as Ipi-L-3 here) is reduced with dithiothreitol (DTT) at a molar ratio of 1:200 for 16 hours under the condition of 50 mM Tris (pH 7.5) and 2 mM EDTA at room temperature. Then it is dialyzed into a buffer of 50 mM Tris (pH 7.5) and 150 mM NaCl. Then, it is re-oxidized with dehydroascorbic acid (DHAA) at a molar ratio of 1:100 and used for coupling with different compounds CP12-CP18 and CP23-CP30 containing different blocking groups and different chemical linkers. The molar ratio of the compound/antibody used for coupling is 1:20.

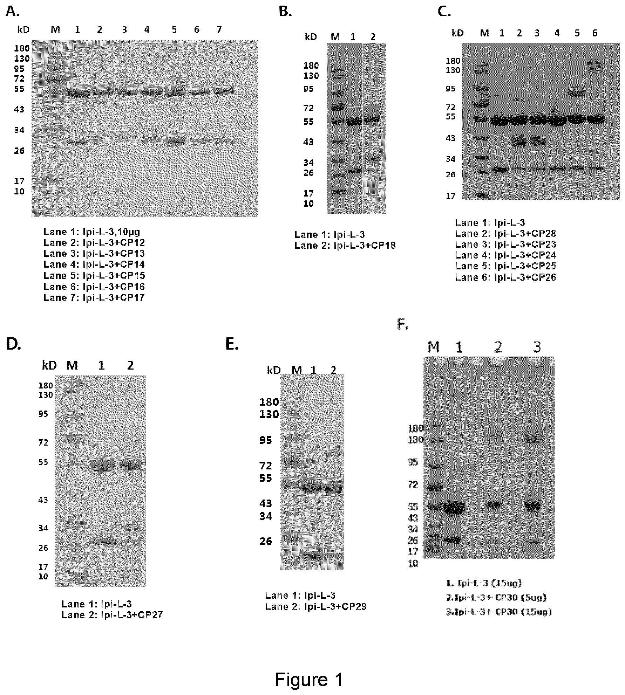

The coupled TMEAbody is further purified by a protein A column to remove excess compounds, and the coupling efficiency is evaluated by SDS-PAGE determination. The results in Figures 1(A-F) indicate that Ipi-L-3 shows high coupling efficiency for these different compounds.

### Example 3: ELISA characterization of Ipi-L-3 TMEAbody.

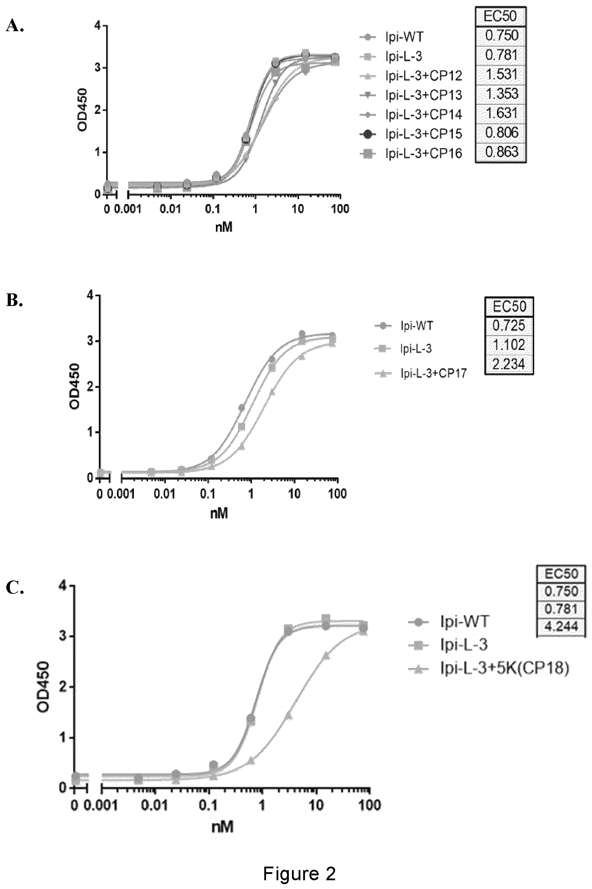

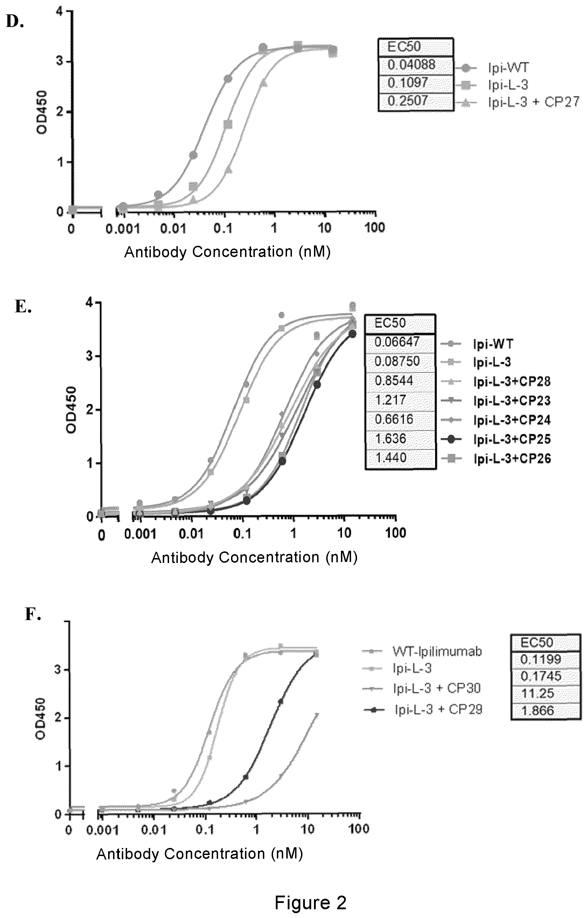

To characterize the binding characteristics of the constructed TMEAbody to human CTLA-4 protein, 0.5 µg/ml human CTLA-4 protein with His tag (Sino Biological, Cat#11159-H08H) was coated on Maxisorp ELISA plates (Nunc) and incubated overnight at 4 °C. Then the plates were washed three times with PBST and blocked with 2% BSA at room temperature for 2 hours. After washing the ELISA plates three times with PBST, the plates were incubated with a series of concentrations of the conjugated TMEAbody, the antibody (in cysteine mutant form) before TMEAbody conjugation, and the control wild-type Ipilimumab antibody for one hour. Then the plates were washed three times with PBST and incubated with anti-human Fc antibody conjugated with HRP (Invitrogen, Cat# A18829) at a dilution of 1:5000 at room temperature for 0.5 hours. After washing three times with PBST, the plates were developed with tetramethylbenzidine (TMB, Solarbio, catalog number PR1200) and ELISA stop buffer (Solarbio, catalog number C1058). Then the absorbance at 450 nm was measured by an ELISA microplate reader (BioTek, ELx800 Absorbance Microplate Reader). Then the data were analyzed by GraphPad Prism 5 software. The ELISA results are shown in Figures 2(A-F). These results indicate that the binding ability of TMEA antibodies to CTLA-4 protein is blocked at different levels by different blocking groups. Generally, the blocking efficiency increases with the size of the blocking group.

### Example 4: Activation of acid-mediated Ipi-L-3 + CP23, Ipi-L-3 + CP24, Ipi-L-3 + CP25 and Ipi-L-3 + CP26 TMEAbody.

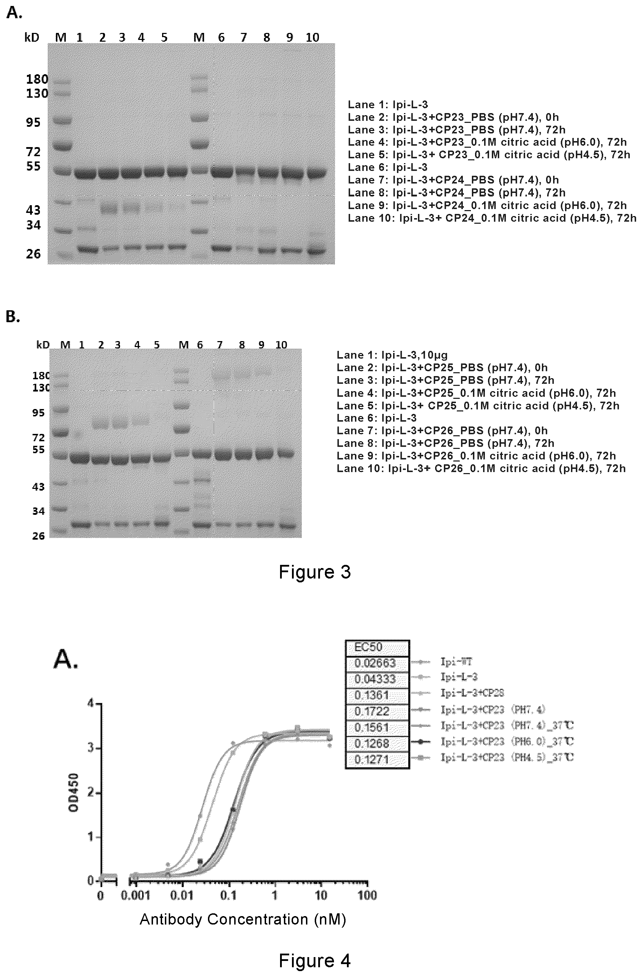

The compounds CP23, CP24, CP25 and CP26 contain hydrazone bonds that are unstable at acidic pH. The Ipi-L-3 TMEAbody conjugated with these compounds was dialyzed into citrate buffers at pH 4.5, pH 5.0 and pH 6.0, and acid-mediated cleavage was monitored by SDS-PAGE. As shown in Figures 3(A-B), all TMEAbodies with these compounds exhibit varying degrees of cleavage, with the extent of cleavage depending on the pH level, and the lower the pH, the higher the cleavage efficiency.

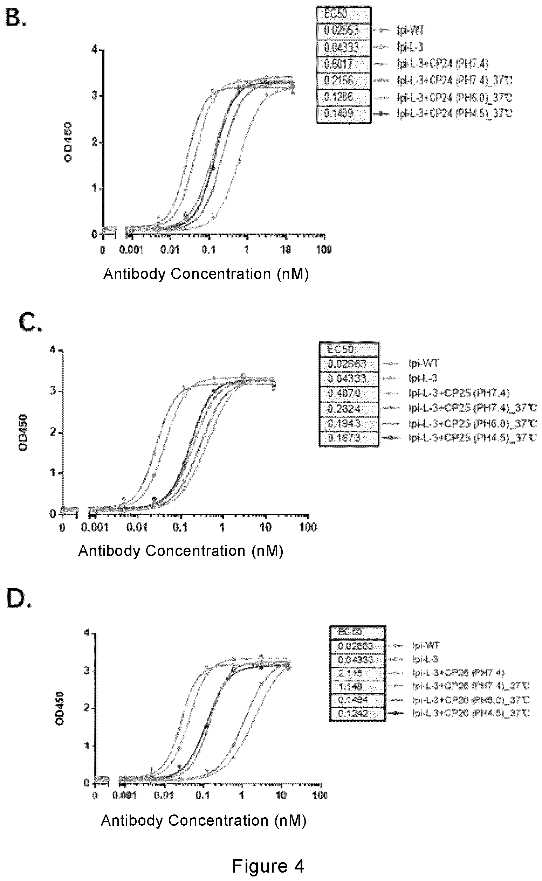

Then, the ELISA assay method from Example 2 was used to evaluate the recovery of binding activity. The results are summarized in Figures 4(A-D). The cleavage mediated by low pH led to a corresponding recovery of activity, with the level of activity recovery being proportional to the level of cleavage.

### Example 5: Activation of Ipi-L-3+CP23 TMEAbody mediated by Legumain

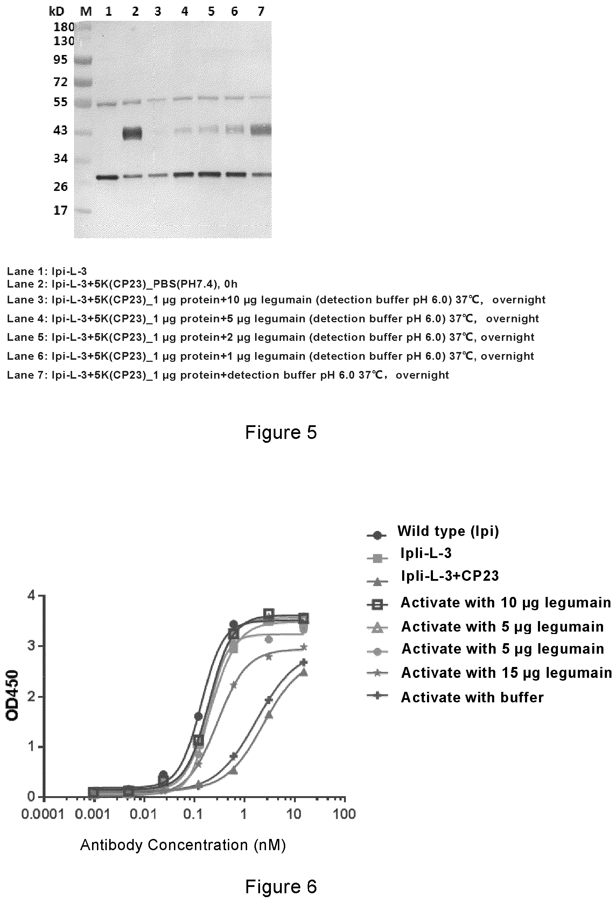

The conjugated Ipi-L-3+CP23 TMEAbody was incubated with activated Legumain in a buffer solution of pH 6.0, 50 mM MES, and 250 mM NaCl at 37°C overnight. The activation efficiency was assessed using a protein blotting method. An anti-human Fab HRP antibody (SIGMA, A0293) was used to detect the light chain of the antibody. As shown in Figure 5, 1µg of Ipi-L-3+CP23 TMEAbody can be activated by 1-5µg of Legumain.

The ELISA assay was then used to test the recovery of binding capacity after Legumain digestion. The results are shown in Figure 6. The results indicate that varying degrees of Legumain cleavage lead to corresponding levels of activity recovery.

### Example 6: ELISA characterization of acid-activation and ELISA characterization of enzyme- activation.

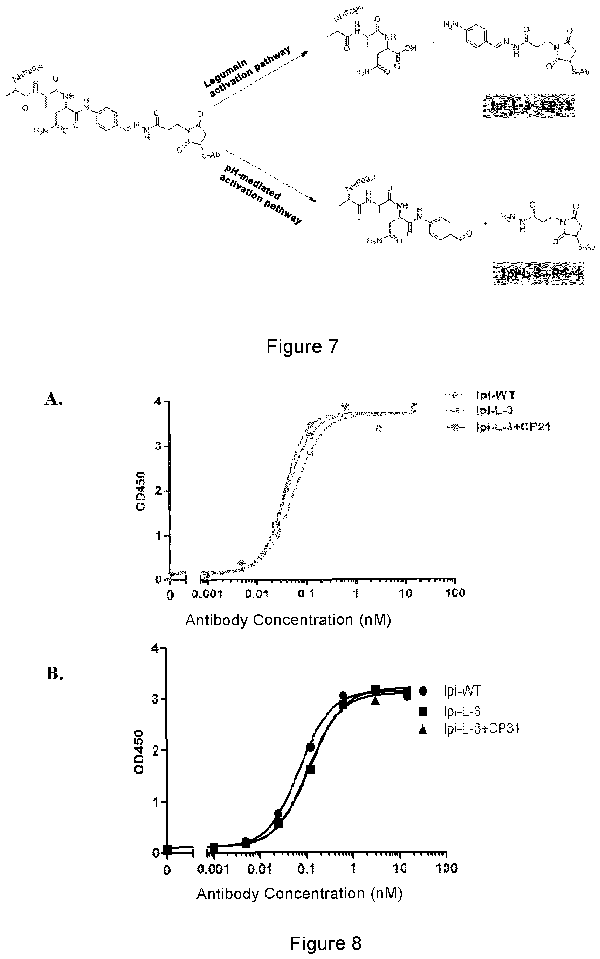

The schematic diagram of legumain activation and acid activation of the TMEAbody conjugated with Ipi-L-3 and CP23 is shown in Figure 7. To test the activity recovery after acid activation, we conjugate R4-4 with Ipi-L-3, and the molecular structure is the same as that after acid-mediated activation.

After conjugation with R4-4, ELISA was used to evaluate the effect on the binding activity of Ipi-L-3 of the small molecule fragment remaining on the antibody after activation. The results are shown in Figure 8(A). After Ipi-L-3 is conjugated with R4-4, the conjugate maintains the binding activity of Ipi-L-3, which means that the structure of Ipi-L-3 + CP23 after acid activation can maintain the same binding activity of the wild-type Ipilimumab antibody.

To test the activity recovery after enzyme activation, we conjugated CP31 with Ipi-L-3, and the molecular structure is the same as that after enzyme-mediated activation. After conjugation with CP31, ELISA was used to evaluate the effect on the binding activity of Ipi-L-3 of the small molecule fragment remaining on the antibody after activation. The results are shown in Figure 8(B). After Ipi-L-3 is conjugated with CP31, the conjugate maintains the binding activity of Ipi-L-3, which means that the structure of Ipi-L-3 + CP23 after Legumain activation can maintain the same binding activity of the wild-type Ipilimumab antibody.

### Example 7: Rapid acid activation and activity recovery assessment of Ipi-L-3 + CP23.

To establish a rapid acid activation method for Ipi-L-3 CP23 TMEAbody, the pH value of the conjugated Ipi-L-3+CP23 TMEAbody was adjusted with HCl to pH 2.0, pH 2.5, and pH 3.5 respectively, and treated for 1 hour, 3 hours, and 5 hours respectively. At the same time, SDS-PAGE and ELISA were used to evaluate the cutting efficiency and activity recovery.

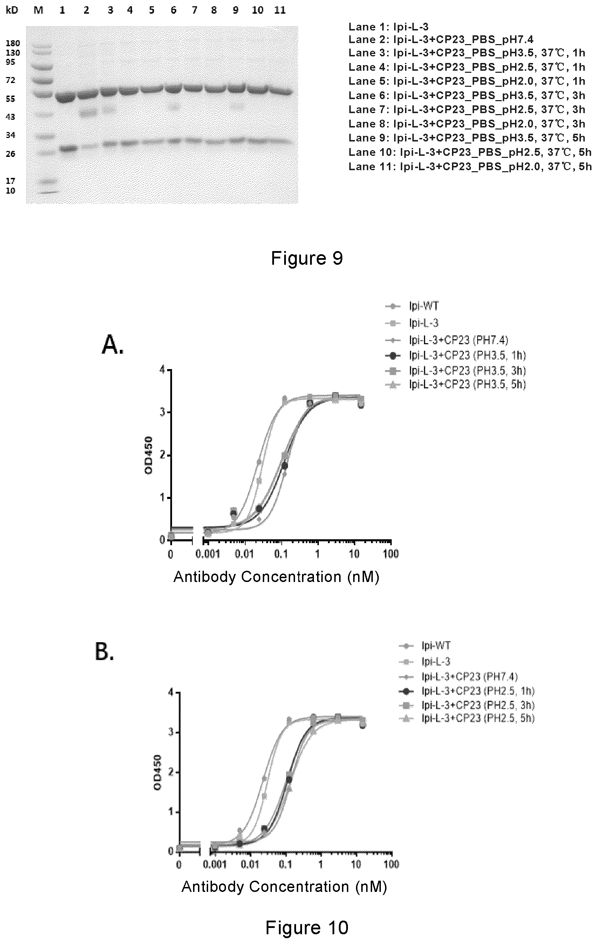

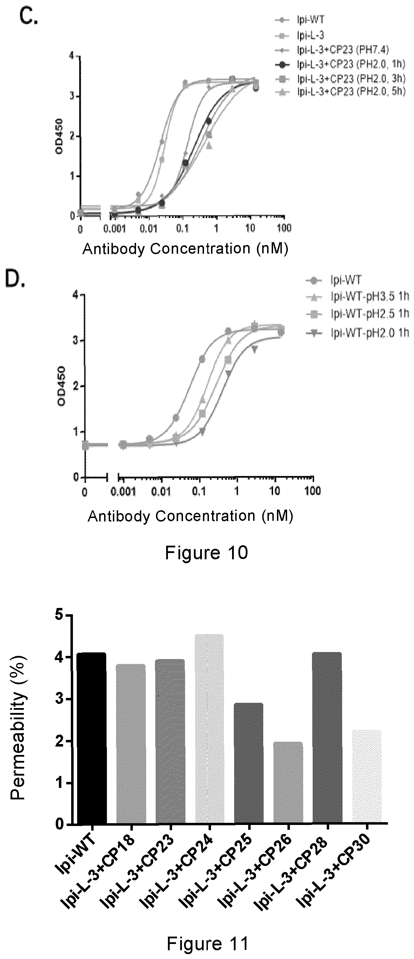

The results are shown in Figures 9 and 10 (A-D). The results show that under extremely low pH conditions, such as at pH 2.0 and pH 2.5, this TMEAbody can be rapidly cleaved. However, the ELISA results show that extremely low pH will lead to a significant loss of antibody activity, which may be due to the extremely low pH destroying the structure of the antibody. In addition, the wild-type Ipilimumab antibody will also significantly loss activity under the conditions of pH 3.5, pH 2.5 and pH 2.0.

### Example 8: Evaluation of the pharmacokinetics and tissue distribution of a series of TMEAbodies in mouse tumor models.

To evaluate the distribution and activation of TMEAbody in vivo, the above series of TMEAbody at a dose of 10 mg/kg was injected into CT26 tumor-bearing Balb/c mice. At different time points afterwards, plasma and different tissues were taken to analyze the concentration of TMEAbody or activated TMEAbody. Tissue homogenates were prepared in 2.5 volumes of PBS, to which 1X Halt^{™} Protease Inhibitor Cocktail (100X) (Thermo Fisher Scientific, catalog number 78430) was added, and the supernatant was collected and used for ELISA detection. For the determination of TMEAbody concentration, 5 µg/ml anti-PEG antibody (Abcam, catalog number ab51257) was used to capture TMEAbody, and the captured TMEAbody was detected with anti-human Fc antibody (Invitrogen, A18829, diluted 1:50,000). Standard curves were plotted using different TMEAbody of series concentrations. The supernatant of plasma and tissue homogenate was diluted to an appropriate multiple for ELISA determination and the concentration was converted using a standard curve. To determine the concentration of active TMEAbody, it was coated with 0.5 µg/ml human CTLA-4 protein (Sino Biological, catalog number 11159-H08H), and wild-type Ipilimumab at series concentrations was used as the primary antibody while anti-human Fc antibody (Invitrogen, catalog number A18829) at a dilution of 1:5000 was used as the secondary antibody, and standard curves were made using different concentrations of Ipilimumab antibody. The supernatant of plasma and tissue homogenate was diluted to an appropriate multiple for ELISA determination and the concentration was converted using a standard curve.

We focus on two important parameters in the PK and tissue distribution data of TMEAbody. The first parameter is the activation rate in tumors and other tissues. A high specific activation rate in tumors rather than in other tissues is preferred because it will produce lower peripheral toxicity. The activation rates of different TMEAbodies in different tissues are shown in Table 3.

**Table 3: Activation efficiency of TMEAbody in different tissues at different times (days).**

| | Activation rate (%) | D0 | D2 | D5 | D9 |
|---|---|---|---|---|---|
| Ipi-L3+CP18 | blood | 4.32 | 11.52 | 22.62 | 30.26 |
| | tumor | ND | 12.69 | 24.54 | 32.15 |
| | heart | ND | 16.32 | 26.51 | 35.47 |
| | lung | ND | 13.54 | 19.85 | 25.65 |
| | kidney | ND | 14.23 | 23.36 | 28.65 |
| | spleen | ND | 15.23 | 33.23 | 46.32 |
| | liver | ND | 16.57 | 37.1 | 45.24 |
| Ipi-L-3+CP23 | blood | 3.29 | 19.48 | 23.9 | 42.66 |
| | tumor | ND | 39.88 | 74.58 | 85.62 |
| | heart | ND | 17.25 | 23.65 | 32.25 |
| | lung | ND | 19.62 | 22.35 | 28.62 |
| | kidney | ND | 23.35 | 23.64 | 31.26 |
| | spleen | ND | 9.24 | 25.32 | 32.63 |
| | liver | ND | 21.67 | 35.34 | 45.63 |
| Ipi-L-3+CP24 | blood | 8.13 | 11.3 | 32.82 | 31.12 |
| | **tumor** | **ND** | **32.42** | **62.19** | **70.25** |
| | heart | ND | 12.25 | 15.32 | 25.63 |
| | lung | ND | 11.36 | 13.69 | 23.35 |
| | kidney | ND | 15.26 | 22.36 | 28.25 |
| | spleen | ND | 16.62 | 22.65 | 30.97 |
| | liver | ND | 18.96 | 30.28 | 46.18 |
| Ipi-L-3+CP25 | blood | 3.67 | 15.17 | 18.25 | 26.36 |
| | **tumor** | **ND** | **28.14** | **56.62** | **65.32** |
| | heart | ND | 9.58 | 11.36 | 22.65 |
| | lung | ND | 11.54 | 15.88 | 20.65 |
| | kidney | ND | 8.53 | 12.32 | 15.23 |
| | spleen | ND | 19.59 | 13.25 | 28.65 |
| | liver | ND | 22.52 | 28.63 | 41.23 |
| | blood | 1.64 | 4.7 | 12.3 | 23.35 |
| Ipi-L-3+CP26 | **tumor** | **ND** | **18.96** | **39.84** | **61.79** |
| | heart | ND | 6.68 | 12.36 | 19.62 |
| | lung | ND | 5.69 | 11.09 | 20.69 |
| | kidney | ND | 6.35 | 16.32 | 25.25 |
| | spleen | ND | 5.46 | 11.32 | 22.64 |
| | liver | ND | 8.69 | 20.63 | 36.49 |
| Ipi-L-3+CP28 | blood | 5.65 | 12.36 | 17.58 | 20.65 |
| | **tumor** | **ND** | **20.07** | **40.26** | **56.32** |
| | heart | ND | 11.29 | 15.65 | 19.87 |
| | lung | ND | 12.79 | 17.56 | 16.09 |
| | kidney | ND | 11.68 | 18.26 | 25.64 |
| | spleen | ND | 13.58 | 14.76 | 21.64 |
| | liver | ND | 16.89 | 20.51 | 32.89 |
| Ipi-L-3+CP30 | blood | 4.38 | 7.89 | 18.29 | 23.01 |
| | **tumor** | **ND** | **15.69** | **22.35** | **32.58** |
| | heart | ND | 8.59 | 10.26 | 13.05 |
| | lung | ND | 9.47 | 12.64 | 14.16 |
| | kidney | ND | 7.56 | 14.23 | 16.02 |
| | spleen | ND | 9.44 | 13.53 | 17.06 |
| | liver | ND | 7.62 | 12.62 | 20.36 |

D0 day is the starting point of drug administration, so ND indicates that there is no data in organs on this day but only data in blood.

As shown in the results in Table 3, Ipi-L-3+CP23/CP24/CP25/CP26/CP28/CP30 exhibit tumor-preferential activation to different extents, and the activation efficiency decreases as the blocking groups increase. Linkers with both legumain and acid activation (CP23, CP24, CP25, CP26) exhibit higher activation efficiency compared to linkers activated only by legumain (CP28, CP30).

Another parameter we focus on is tumor permeability because PEG will increase the size of the antibody and may affect the tumor permeability of the antibody. We use the ratio of tumor AUC/blood AUC (total concentration of activated and unactivated TMEAbody TMEABODY) to represent permeability. As shown in Figure 11, PEG with a molecular weight greater than 20 kD will significantly affect the permeability of the antibody.

### Example 9: Therapeutic effect of Ipi-L-3 TMEAbody in the MC38 tumor model of hCTLA-4 gene knock-in mice.

To evaluate the in vivo efficacy of Ipi-L-3 TMEAbody in treating mouse tumors, Ipi-L-3+CP23 TMEAbody, wild-type Ipilimumab, and control human IgG were administered to the MC38 colon adenocarcinoma tumor model of human CTLA-4 gene knock-in C57BL/6 mice (Shanghai Model Organisms Center). In C57BL/6 mice with human CTLA-4 gene knock-in, 2E6 MC38 cells were subcutaneously injected into the left lower abdominal quadrant. Seven days after tumor growth, the animals were grouped according to the same average tumor volume. Animals were injected twice a week with the specified dose of control human IgG, wild-type Ipilimumab or Ipi-L-3 + CP23 TMEAbody (antibody concentration without PEG molecular weight, n = 6), and the tumor volume of each animal was monitored.

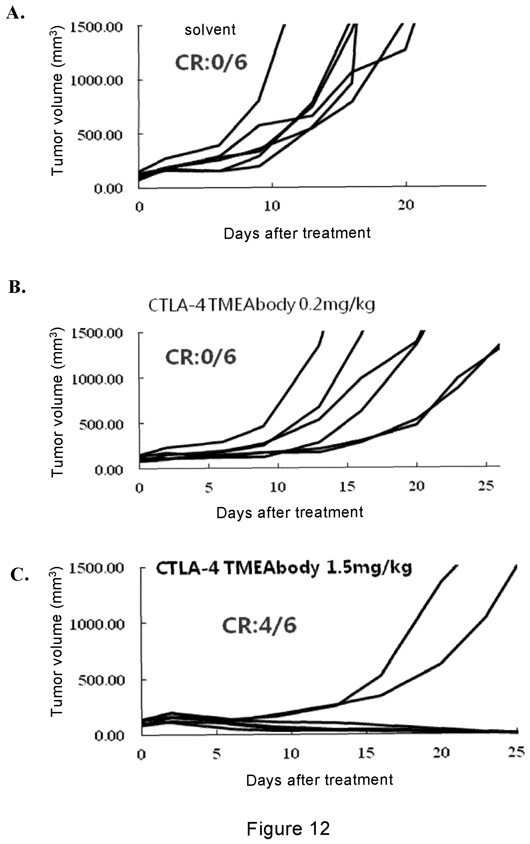

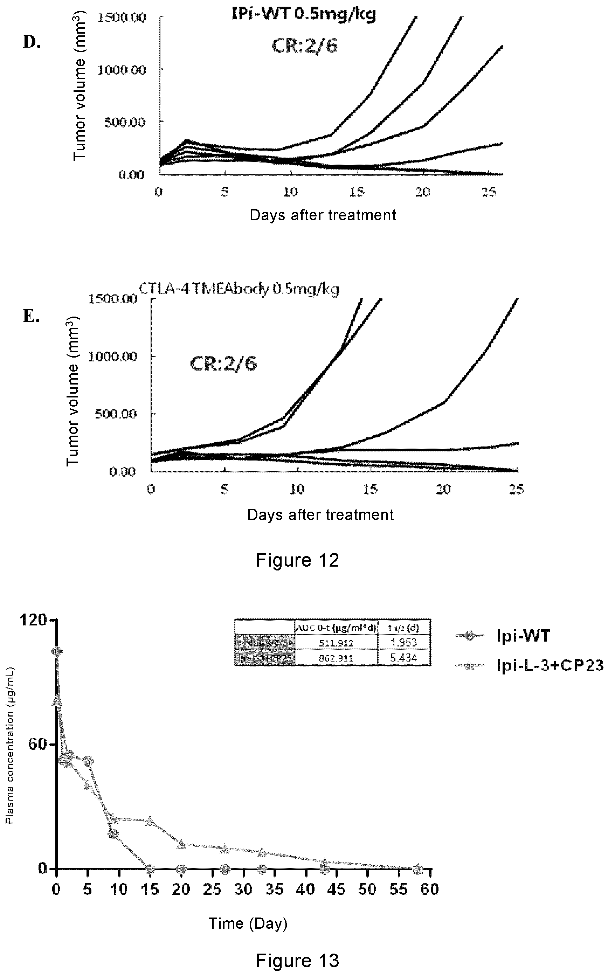

The results are shown in Figure 12 (A-E). Ipi-L-3+CP23 TMEAbody shows a dose-dependent tumor growth inhibitory effect. At a dose of 0.5 mg/kg, Ipi-L-3+CP23 TMEAbody shows the same complete remission rate (CR) as the WT Ipilimumab antibody.

### Example 10: Ipi-L-3 TMEAbody shows an extended serum half-life in human CTLA-4 gene knock-in mice.

Antibodies show target antigen-mediated clearance in vivo, and we speculate that since TMEAbody has a decreased binding force to the target antigen, the target antigen-mediated clearance effect will be inhibited, thereby prolonging its half-life. hCTLA-4 knock-in mice (Shanghai Model Organisms Center) were injected with wild-type Ipilimumab antibody or Ipi-L-3 + CP23 TMEAbody, and plasma was collected at different time points, and the antibody concentration was determined by a "sandwich"-type ELISA method using anti-human Fc and anti-human Fab antibodies. As shown in Figure 13, compared with wild-type Ipilimumab antibody, Ipi-L-3 + CP23 TMEAbody shows an extended serum half-life and AUC.

### Example 11: Ipi-L-3 TMEAbody shows a reduced peripheral T cell activation effect.

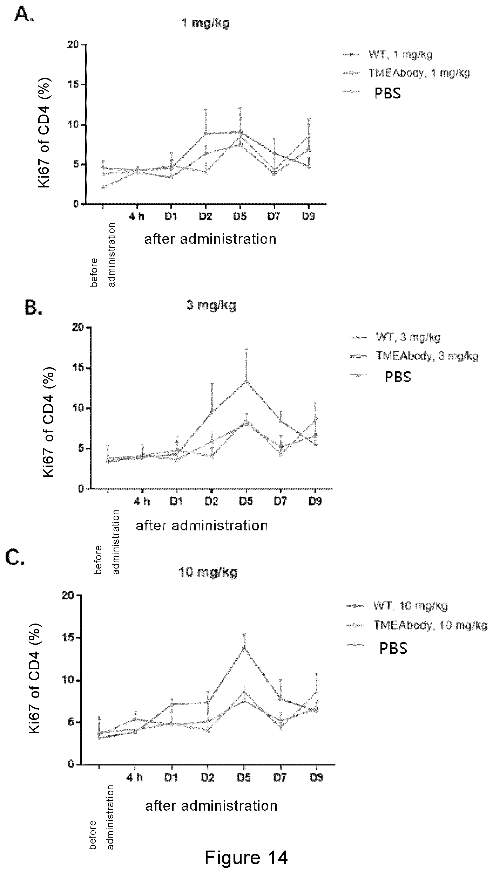

To evaluate the activation effect of wild-type Ipilimumab antibody and Ipi-L-3+CP23 TMEAbody on peripheral CD4+ T cells, wild-type Ipilimumab, Ipi-L-3+CP23 TMEAbody or PBS was injected into hCTLA-4 knock-in mice (Shanghai Model Organisms Center) at doses of 1 mg/kg, 3 mg/kg, and 10 mg/kg respectively. At the specified time points, blood was collected and Ki67 of CD4+ T cells was monitored as a proliferation marker. As shown in Figure 14 (A-C), Ipi-L-3 + CP23 TMEAbody did not stimulate the proliferation of CD4+ T cells at doses of 3 mg/kg and 10 mg/kg, indicating that TMEAbody has a low peripheral blood T cell activation effect due to its blocked activity, suggesting that it has lower immune-related toxicity.

### Example 12: Ipi-L-3 TMEAbody causes a lower GvHD effect in NSG mice implanted with human PBMC

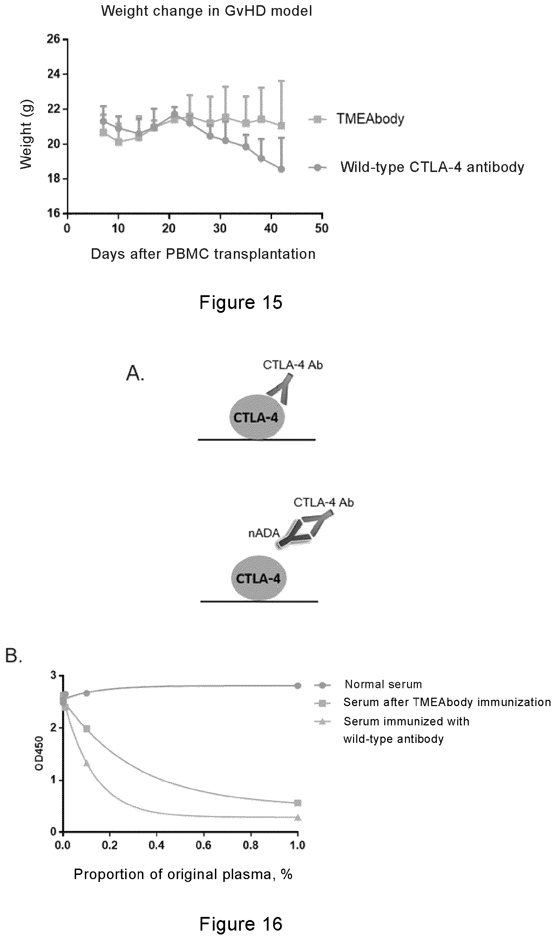

To further evaluate the immunotoxicity of Ipilimumab and Ipi-L-3 TMEAbody, wild-type Ipilimumab and Ipi-L-3 + CP23 TMEAbody were administered to NSG immunodeficient mice transplanted with human PBMC (Shanghai Model Organisms Center). Human PBMC cells of 5×10⁶ were transplanted into NSG mice through the tail vein and after 10 days of reconstruction 10 mg/kg Ipilimumab or Ipi-L-3 TMEAbody was administered twice a week. Observe animal weight as an indicator of graft-versus-host disease (GvHD effect). As can be seen from Figure 15, wild-type Ipilimumab significantly aggravates GvHD, leading to a sharp drop in body weight, while Ipi-L-3+CP23 TMEAbody does not significantly aggravate GvHD, suggesting that Ipi-L-3+CP23 reduces the activation of toxic T cells in healthy tissues.

### Example 13: Ipi-L-3 TMEAbody causes lower immunogenicity in mice

To evaluate the immunogenicity of Ipi-L-3 TMEAbody, Ipi-L-3+CP23 TMEAbody and WT CTLA-4 antibody were injected into Balb/c mice for immunization, and PBS was set as a negative control. For the initial immunization, 50 µg of Ipi-L-3+CP23 TMEAbody or wild-type Ipilimumab antibody was mixed with 1:1 complete Freund's adjuvant (CFA) and injected intraperitoneally into mice, with 5 mice in each group. To further enhance the immunity, 14 days after the initial immunization, 25 µg of Ipi-L-3 TMEAbody or wild-type Ipilimumab antibody was mixed with 1:1 incomplete Freund's adjuvant and injected intraperitoneally into mice. Plasma was collected 21 days after the initial immunization and immunogenicity was analyzed by ELISA. 0.5 µg/ml human CTLA-4-His protein (Sino Biological, Cat#11159-H08H) was coated on ELISA plates (BeaverBio, Cat#REF40301) overnight. After blocking with 1% BSA, a series of concentrations of serum were mixed with 0.1078 µg/ml wild-type Ipilimumab antibody, and then 100 µl of the mixture was added to the wells of a 96-well ELISA plate fixed with human CTLA-4-his protein. Peroxidase-conjugated goat anti-human IgG (Fab specific) antibody (Sigma, Cat#A0293) at 1:5000 was used as the secondary antibody, and the plate was developed with tetramethylbenzidine (TMB, Solarbio, catalog number PR1200) and ELISA stop buffer. The absorbance at 450 nm was measured with an ELISA plate reader (Biotek, ELx800 Absorbance Microplate reader). Data was analyzed by GraphPad Prism 5 software.

The ELISA format is shown in Figure 16(A) of the attached figure and the results are shown in Figure 16(B). These results indicate that Ipi-L-3 TMEAbody has a lower risk of inducing neutralizing anti-drug antibodies (nADA) compared to wild-type Ipilimumab antibody.

### Example 14: Evaluate the therapeutic effect of Ipi-L-3 TMEAbody on the MC38 tumor model of human CTLA-4 gene knock-in mice by FACS.

To evaluate the depletion effect of Treg cells in tumors and periphery of mice after treatment with Ipi-L-3 TMEAbody, Ipi-L-3 + CP23 TMEAbody and Ipilimumab antibody were administered to human CTLA-4 gene knock-in C57 mice carrying MC38 tumors. 7 days after administration, spleens and tumors were collected for FACS analysis (Beckman Coulter, CytoFLEX) of Treg subsets (CD4 + CD25 + FoxP3 +). Then the data was analyzed by GraphPad Prism 5 software.

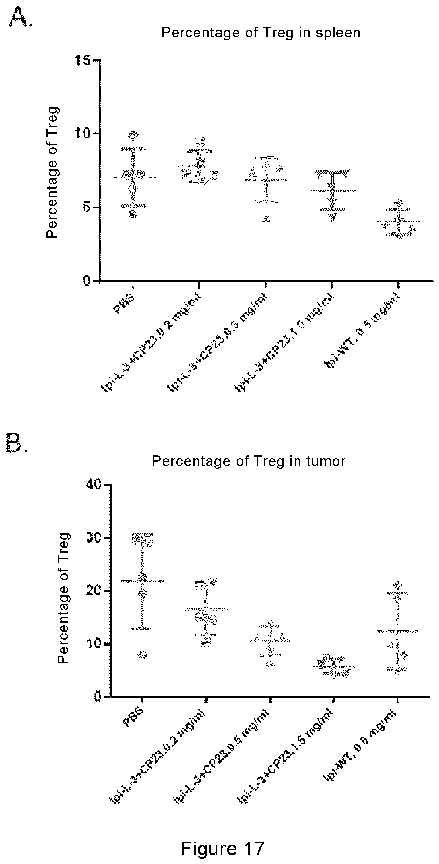

The results are shown in Figures 17(A-B). These results indicate that Ipi-L-3 TMEAbody reduces the percentage of Treg cells in tumors rather than in the spleen, suggesting that it activates locally in tumors to exert anti-tumor efficacy, while having a lower activation in peripheral tissues.

## Claims

1. A conjugate of an anti-CTLA-4 antibody having the following structure:
R1-R2-R3-L-R4-S-cys-R5
wherein,
R5 represents an anti-CTLA-4 antibody in which one or more amino acid residues are mutated to cysteine;
cys represents the cysteine residue;
S represents the S atom in the cysteine residue;
R1 is a functional group that blocks the binding of R5 to its antigen, ligand or receptor;
R2 is a linker that can be hydrolyzed and cleaved by one or more proteases in the pathological microenvironment;
R3 is a linker that provides space for protease cleavage;
L is a chemical bond activated under acidic conditions in the pathological microenvironment; preferably, L is a hydrazone bond.
R4 is a group covalently linked to R5 through the sulfur atom on cysteine in R5, which can restore, maintain or improve the binding ability of R5 to its antigen, ligand and receptor after R1-R2-R3-L- is cleaved.

2. The conjugate according to claim 1, wherein, R1 is selected from the group consisting of: NRₐR_{b}-R-CO-, C₁₋₄ alkoxy-(C₁₋₄ alkoxy)ₙ-carbonyl-C₁₋₄ alkylene-carbonyl- 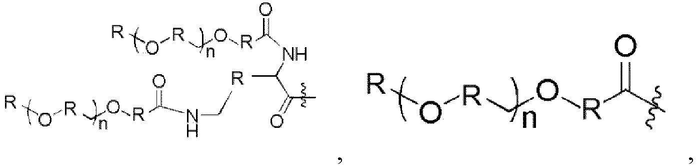 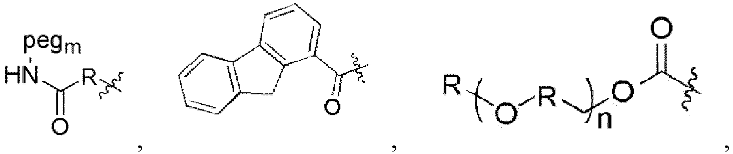 wherein:
each R is independently a C1-4 alkyl group;
each n is independently an integer within the range of 1 to 30000;
pegₘ is polyethylene glycol with a molecular weight of 44 to 132000, wherein m represents the molecular weight of polyethylene glycol;
Rₐ is C₁₋₄ alkyl-O-[(CH₂)_{q}-O]ₙ-(CH₂)_{q}-;
R_{b} is C₁₋₄ alkyl-O-[(CH₂)_{q}-O]ₙ-(CH₂)_{q}-C(O)-;
q is an integer from 1 to 4;
the wavy line indicates the position where R1 is linked to R2;
preferably, the NRₐR_{b}-R-CO- is: 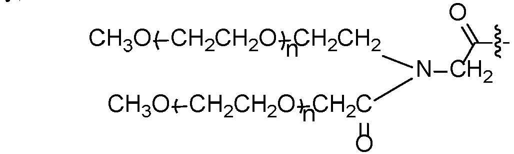 the C₁₋₄ alkoxy-(C₁₋₄ alkoxy) ₙ-carbonyl-C₁₋₄ alkylene-carbonyl- is 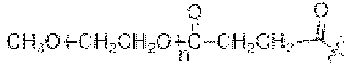
preferably, R1 is selected from the group consisting of: 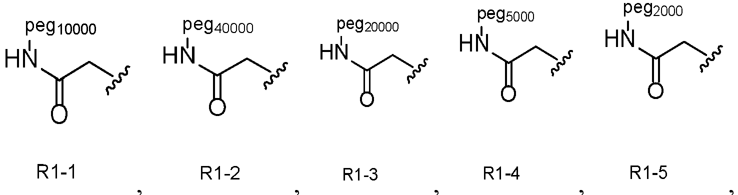 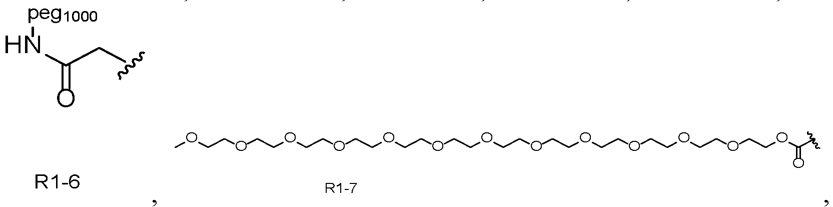 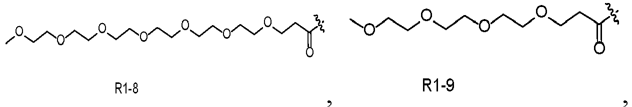 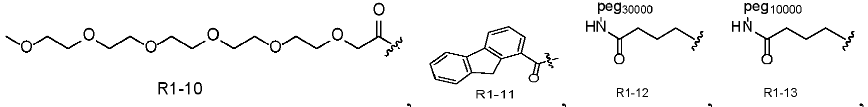 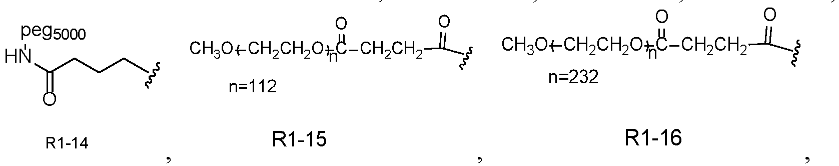 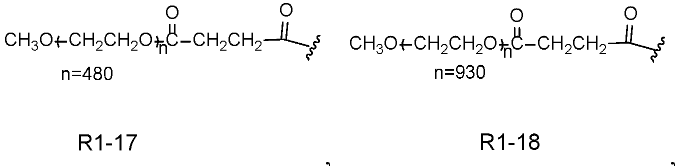 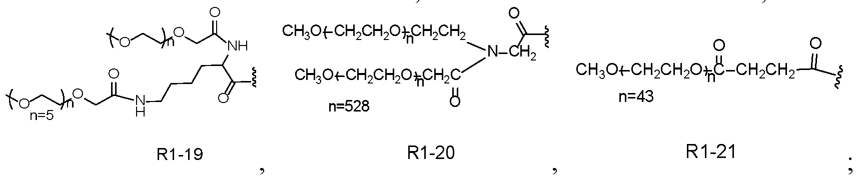
more preferably, R1 is R1-7, R1-15, R1-16, R1-17, R1-20 or R1-21.

3. The conjugate according to claim 1, wherein R2 is a tripeptide, wherein amino acid residue in the tripeptide linked to R1 is selected from the group consisting of Ala, Thr, Val and Ile, the intermediate amino acid residue is selected from the group consisting of Ala, Thr, Val and Asn, and the amino acid residue linked to R3 is selected from Asn and Asp; preferably, R2 is Ala-Ala-Asn or Ala-Ala-Asp;
wherein, R2 is linked to R1 through the amino group of its amino acid residue by an amide bond, ester bond, carbamate bond, urea bond or hydrazone bond, and is linked to R3 through the carboxyl group of its amino acid residue by an amide bond, ester bond, carbamate bond, urea bond or hydrazone bond.

4. The conjugate according to claim 1, wherein R3 is -HN-phenyl-R'-, wherein R' is a bond; preferably, the NH group and R' are located at the para position of the phenyl group.

5. The conjugate according to claim 1, wherein the structure of R1-R2-R3-L-R4 is represented as: 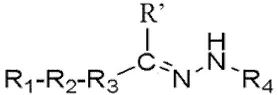 wherein, R' is H or C1-4 alkyl.

6. The conjugate according to claim 1, wherein R4 is as shown in the following formula: 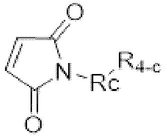
wherein, Rc is selected from the group consisting of: C1-12 alkylene, C1-12 alkylene-O-C1-12 alkylene, C1-12 alkylene-C3-8 cycloalkyl, (C1-4 alkylene-O)p-C1-12 alkylene, C1-12 alkylene-carbonylamino-(C1-4 alkylene-O)p-C1-12 alkylene, -phenyl-C1-12 alkylene, C3-8 cycloalkyl, C1-12 alkylene-C3-8 cycloalkyl-C1-12 alkylene, C1-12 alkylene-NHCO-O-C1-12 alkylene, C1-12 alkylene-COO- and C1-12 alkylene-phenyl-C1-12 alkylene; p is an integer from 1 to 10, preferably is 1, 2, 3 or 4;
R_{4-c} is a bond or -CO-;
wherein, the L group is linked to the R4 group through R_{4-c} of the R4 group; the R4 group is linked to the S atom of the cysteine of R5 through maleimide;
preferably, Rc is C1-12 alkylene and R_{4-c} is -CO-.

7. The conjugate according to claim 1, wherein the anti-CTLA-4 antibody is Ipilimumab;
preferably, the mutation positions in the heavy chain of Ipilimumab are selected from the group consisting of: Gln3, Arg19, Leu20, Ser25, Gly26, Phe27, Thr28, Phe29, Ser30, Ser31, Tyr32, Thr33, Met34, His35, Gly44, Phe50, Ile51, Ser52, Tyr53, Asp54, Gly55, Asn56, Asn57, Lys58, Tyr59, Tyr60, Ala61, Asp62, Ser63, Lys65, Gly66, Thr69, Ser71, Arg72, Asp73, Asn74, Ser75, Lys76, Asn77, Thr99, Gly100, Trp101, Leu102, Gly103, Pro104, Leu105, Asp106 and Tyr107; preferably selected from the group consisting of: Ser30, Ser31, Tyr32, Thr33, Ile51, Asp54, Gly55, Asn56, Lys58, Tyr59, Tyr60, Ala61, Asp62, Ser63, Lys65, Gly66, Gly100, Trp101, Leu102, Gly103, Pro104, Leu105, Asp106 and Tyr107.
preferably, the mutation positions in the light chain of Ipilimumab are selected from the group consisting of: Gln6, Arg24, Ala25, Ser26, Gln27, Ser28, Val29, Gly30, Ser31, Ser32, Tyr33, Leu34, Ala35, Tyr37, Ile49, Tyr50, Gly51, Ala52, Phe53, Ser54, Arg55, Ala56, Thr57, Gly58, Ile59, Pro60, Asp61, Arg62, Ser68, Gly69, Thr70, Gln90, Gln91, Tyr92, Gly93, Ser94, Ser95, Pro96, Trp97, Thr98, Phe99 and Gly100; preferably selected from the group consisting of: Ala25, Ser26, Ser28, Gly30, Ser31, Ser32, Leu34, Ala35, Gly51, Ala52, Ser54, Ala56, Thr57, Gly58, Ile59, Gly93, Ser94, Ser95, Thr98 and Gly100;
the above position numbers refer to SEQ ID NO: 1 and 2;
preferably, Tyr50 in the light chain of Ipilimumab is mutated to C.

8. The conjugate according to claim 1, wherein R1-R2-R3-L-R4 is selected from the group consisting of: 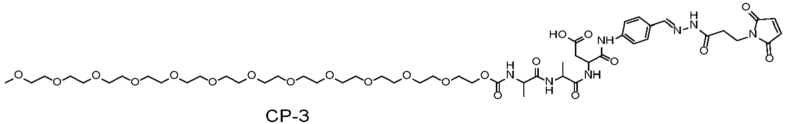 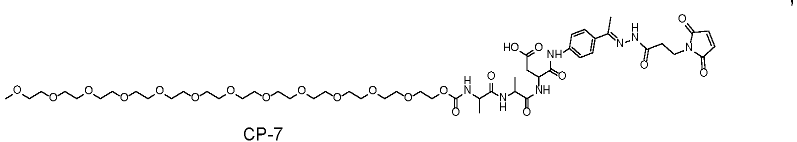 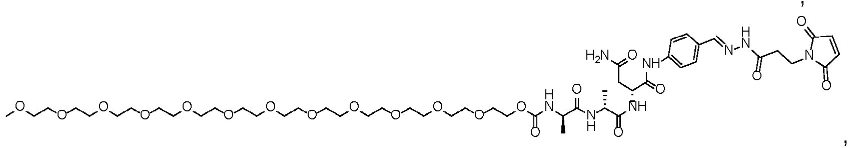 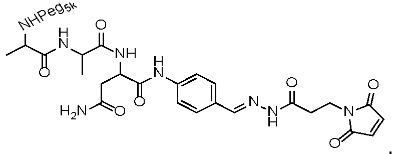 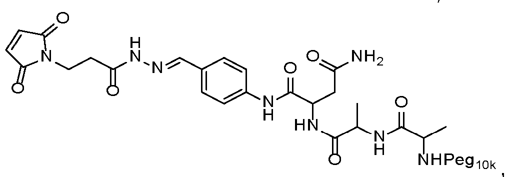 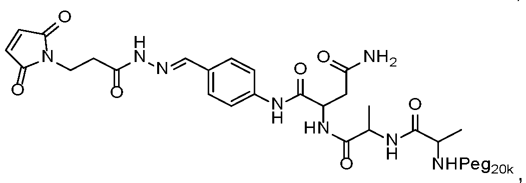 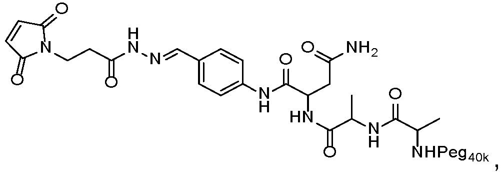 and

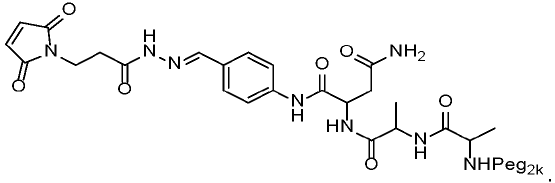

9. A pharmaceutical composition, wherein the pharmaceutical composition contains the conjugate according to any one of claims 1 to 8 and a pharmaceutically acceptable carrier.

10. Use of the conjugate according to any one of claims 1 to 8 in the manufacture of a medicament for treating or preventing tumors or inflammations; preferably, the tumor or inflammation is a tumor or inflammation that can be treated or prevented by an anti-CTLA-4 antibody; more preferably, the tumor is selected from the group consisting of: bladder cancer, brain cancer, breast cancer, cervical cancer, colorectal cancer, esophageal cancer, kidney cancer, liver cancer, lung cancer, nasopharyngeal cancer, pancreatic cancer, prostate cancer, skin cancer, gastric cancer, uterine cancer, ovarian cancer, testicular cancer and blood cancer.
